# EUROPEAN PATENT SPECIFICATION

(11) **EP 3 008 175 B1**
(45) Date of publication and mention of the grant of the patent: **07.08.2019**
(21) Application number: 14810447.4
(22) Date of filing: 15.06.2014
(51) Int. Cl.: A61K 35/76, A61K 38/16, A61K 35/768, A61P 37/00, A61K 39/12

(54) **IMMUNOSUPPRESSIVE VIRAL LIKE PARTICLES BASED ON GAMMARETROVIRUS**
IMMUNSUPPRESSIVE VIRUSÄHNLICHE PARTIKEL VON GAMMARETROVIREN
PARTICULES IMMUNOSUPPRESSIVES DE TYPE VIRAL BASÉES SUR UN GAMMARÉTROVIRUS

(30) Priority: 15.06.2013 US 201361835545 P
(43) Date of publication of application: 20.04.2016
(73) Proprietor: Tocagen Inc., San Diego, CA 92109 (US)
(72) Inventor: GRUBER, Harry, E., Rancho Santa Fe, CA 92067 (US); JOLLY, Douglas, J., Encinitas, CA 92024 (US); LIN, Amy, H., San Diego, CA 92130 (US)
(74) Representative: Gill Jennings & Every LLP
(86) International application number: PCT/US2014/042444
(87) International publication number: WO 2014/201449

(56) References cited:
- WO-A1-2012/162367
- WO-A1-2015/148683
- WO-A2-2007/107156
- WO-A2-2010/105251
- US-A1- 2011 136 208
- DATABASE BIOSIS [Online] BIOSCIENCES INFORMATION SERVICE, PHILADELPHIA, PA, US; April 2012 (2012-04), TAO LING ET AL: "H5N1 influenza virus-like particles produced by transient expression in mammalian cells induce humoral and cellular immune responses in mice", XP002763254, Database accession no. PREV201200354570 & CANADIAN JOURNAL OF MICROBIOLOGY, vol. 58, no. 4, April 2012 (2012-04), pages 391-401, ISSN: 0008-4166(print), DOI: 10.1139/W2012-006
- KADER MUHAMUDA ET AL: "Blocking TLR7-and TLR9-mediated IFN-alpha Production by Plasmacytoid Dendritic Cells Does Not Diminish Immune Activation in Early SIV Infection", PLOS PATHOGENS, vol. 9, no. 7, July 2013 (2013-07), XP002763255,
- OMAR D PEREZ ET AL: "Design and Selection of Toca 511 for Clinical Use: Modified Retroviral Replicating Vector With Improved Stability and Gene Expression", MOLECULAR THERAPY, vol. 20, no. 9, 1 May 2012 (2012-05-01), pages 1689-1698, XP055077731, ISSN: 1525-0016, DOI: 10.1038/mt.2012.83
- LIU, W ET AL.: 'Inibition Of Interferon Signaling By The New York 99 Strain And Kunjin Subtype Of West Nile Virus Involves Blockage Of STAT1 And STAT2 Activation By Nonstructural Proteins.' JOURNAL OF VIROLOGY. vol. 79, no. 3, February 2005, pages 1934 - 1942, XP055299160
- KEKARAINEN, T ET AL.: 'Porcine Circovirus Type 2 (PCV2) Viral Components Immunomodulate Recall Antigen Responses.' VETERINARY IMINONOLOGY AND IMMUNOPATHOLOGY. vol. 124, no. 1-2;, 15 July 2008, pages 41 - 49, XP022691070

### SEQUENCE LISTING

<110> Tocagen Inc.
<120> IMMUNOSUPPRESSIVE COMPONENTS ASSOCIATED WITH RETROVIRAL REPLICATING VECTORS
<130> JWJ02188EP
<140> 14810447.4
   <141> 2014-06-15
<150> PCT/US2014/042444
   <151> 2014-06-15
<150> US 61/835,545
   <151> 2013-06-15
<160> 4
<170> PatentIn version 3.5
<210> 1
   <211> 5277
   <212> DNA
   <213> Murine leukemia virus
<220>
   <221> CDS
   <222> (1)..(5277)
<400> 1
   atg ggc cag act gtt acc act ccc tta agt ttg acc tta ggt cac tgg        48
   Met Gly Gln Thr Val Thr Thr Pro Leu Ser Leu Thr Leu Gly His Trp
   1               5                   10                  15
   aaa gat gtc gag cgg atc gct cac aac cag tcg gta gat gtc aag aag        96
   Lys Asp Val Glu Arg Ile Ala His Asn Gln Ser Val Asp Val Lys Lys
               20                  25                  30
   aga cgt tgg gtt acc ttc tgc tct gca gaa tgg cca acc ttt aac gtc       144
   Arg Arg Trp Val Thr Phe Cys Ser Ala Glu Trp Pro Thr Phe Asn Val
           35                  40                  45
   gga tgg ccg cga gac ggc acc ttt aac cga gac ctc atc acc cag gtt       192
   Gly Trp Pro Arg Asp Gly Thr Phe Asn Arg Asp Leu Ile Thr Gln Val
       50                  55                  60
   aag atc aag gtc ttt tca cct ggc ccg cat gga cac cca gac cag gtc       240
   Lys Ile Lys Val Phe Ser Pro Gly Pro His Gly His Pro Asp Gln Val
   65                  70                  75                  80
   ccc tac atc gtg acc tgg gaa gcc ttg gct ttt gac ccc cct ccc tgg       288
   Pro Tyr Ile Val Thr Trp Glu Ala Leu Ala Phe Asp Pro Pro Pro Trp
                   85                  90                  95
   gtc aag ccc ttt gta cac cct aag cct ccg cct cct ctt cct cca tcc       336
   Val Lys Pro Phe Val His Pro Lys Pro Pro Pro Pro Leu Pro Pro Ser
               100                 105                 110
   gcc ccg tct ctc ccc ctt gaa cct cct cgt tcg acc ccg cct cga tcc       384
   Ala Pro Ser Leu Pro Leu Glu Pro Pro Arg Ser Thr Pro Pro Arg Ser
           115                 120                 125
   tcc ctt tat cca gcc ctc act cct tct cta ggc gcc aaa cct aaa cct         432
   Ser Leu Tyr Pro Ala Leu Thr Pro Ser Leu Gly Ala Lys Pro Lys Pro
       130                 135                 140
   caa gtt ctt tct gac agt ggg ggg ccg ctc atc gac cta ctt aca gaa         480
   Gln Val Leu Ser Asp Ser Gly Gly Pro Leu Ile Asp Leu Leu Thr Glu
   145                 150                 155                 160
   gac ccc ccg cct tat agg gac cca aga cca ccc cct tcc gac agg gac         528
   Asp Pro Pro Pro Tyr Arg Asp Pro Arg Pro Pro Pro Ser Asp Arg Asp
                   165                 170                 175
   gga aat ggt gga gaa gcg acc cct gcg gga gag gca ccg gac ccc tcc         576
   Gly Asn Gly Gly Glu Ala Thr Pro Ala Gly Glu Ala Pro Asp Pro Ser
               180                 185                 190
   cca atg gca tct cgc cta cgt ggg aga cgg gag ccc cct gtg gcc gac         624
   Pro Met Ala Ser Arg Leu Arg Gly Arg Arg Glu Pro Pro Val Ala Asp
           195                 200                 205
   tcc act acc tcg cag gca ttc ccc ctc cgc gca gga gga aac gga cag         672
   Ser Thr Thr Ser Gln Ala Phe Pro Leu Arg Ala Gly Gly Asn Gly Gln
       210                 215                 220
   ctt caa tac tgg ccg ttc tcc tct tct gac ctt tac aac tgg aaa aat         720
   Leu Gln Tyr Trp Pro Phe Ser Ser Ser Asp Leu Tyr Asn Trp Lys Asn
   225                 230                 235                 240
   aat aac cct tct ttt tct gaa gat cca ggt aaa ctg aca gct ctg atc         768
   Asn Asn Pro Ser Phe Ser Glu Asp Pro Gly Lys Leu Thr Ala Leu Ile
                   245                 250                 255
   gag tct gtt ctc atc acc cat cag ccc acc tgg gac gac tgt cag cag         816
   Glu Ser Val Leu Ile Thr His Gln Pro Thr Trp Asp Asp Cys Gln Gln
               260                 265                 270
   ctg ttg ggg act ctg ctg acc gga gaa gaa aaa caa cgg gtg ctc tta         864
   Leu Leu Gly Thr Leu Leu Thr Gly Glu Glu Lys Gln Arg Val Leu Leu
           275                 280                 285
   gag gct aga aag gcg gtg cgg ggc gat gat ggg cgc ccc act caa ctg         912
   Glu Ala Arg Lys Ala Val Arg Gly Asp Asp Gly Arg Pro Thr Gln Leu
       290                 295                 300
   ccc aat gaa gtc gat gcc gct ttt ccc ctc gag cgc cca gac tgg gat         960
   Pro Asn Glu Val Asp Ala Ala Phe Pro Leu Glu Arg Pro Asp Trp Asp
   305                 310                 315                 320
   tac acc acc cag gca ggt agg aac cac cta gtc cac tat cgc cag ttg        1008
   Tyr Thr Thr Gln Ala Gly Arg Asn His Leu Val His Tyr Arg Gln Leu
                   325                 330                 335
   ctc cta gcg ggt ctc caa aac gcg ggc aga agc ccc acc aat ttg gcc        1056
   Leu Leu Ala Gly Leu Gln Asn Ala Gly Arg Ser Pro Thr Asn Leu Ala
               340                 345                 350
   aag gta aaa gga ata aca caa ggg ccc aat gag tct ccc tcg gcc ttc        1104
   Lys Val Lys Gly Ile Thr Gln Gly Pro Asn Glu Ser Pro Ser Ala Phe
           355                 360                 365
   cta gag aga ctt aag gaa gcc tat cgc agg tac act cct tat gac cct        1152
   Leu Glu Arg Leu Lys Glu Ala Tyr Arg Arg Tyr Thr Pro Tyr Asp Pro
       370                 375                 380
   gag gac cca ggg caa gaa act aat gtg tct atg tct ttc att tgg cag        1200
   Glu Asp Pro Gly Gln Glu Thr Asn Val Ser Met Ser Phe Ile Trp Gln
   385                 390                 395                 400
   tct gcc cca gac att ggg aga aag tta gag agg tta gaa gat tta aaa        1248
   Ser Ala Pro Asp Ile Gly Arg Lys Leu Glu Arg Leu Glu Asp Leu Lys
                   405                 410                 415
   aac aag acg ctt gga gat ttg gtt aga gag gca gaa aag atc ttt aat        1296
   Asn Lys Thr Leu Gly Asp Leu Val Arg Glu Ala Glu Lys Ile Phe Asn
               420                 425                 430
   aaa cga gaa acc ccg gaa gaa aga gag gaa cgt atc agg aga gaa aca        1344
   Lys Arg Glu Thr Pro Glu Glu Arg Glu Glu Arg Ile Arg Arg Glu Thr
           435                 440                 445
   gag gaa aaa gaa gaa cgc cgt agg aca gag gat gag cag aaa gag aaa        1392
   Glu Glu Lys Glu Glu Arg Arg Arg Thr Glu Asp Glu Gln Lys Glu Lys
       450                 455                 460
   gaa aga gat cgt agg aga cat aga gag atg agc aag cta ttg gcc act        1440
   Glu Arg Asp Arg Arg Arg His Arg Glu Met Ser Lys Leu Leu Ala Thr
   465                 470                 475                 480
   gtc gtt agt gga cag aaa cag gat aga cag gga gga gaa cga agg agg        1488
   Val Val Ser Gly Gln Lys Gln Asp Arg Gln Gly Gly Glu Arg Arg Arg
                   485                 490                 495
   tcc caa ctc gat cgc gac cag tgt gcc tac tgc aaa gaa aag ggg cac        1536
   Ser Gln Leu Asp Arg Asp Gln Cys Ala Tyr Cys Lys Glu Lys Gly His
               500                 505                 510
   tgg gct aaa gat tgt ccc aag aaa cca cga gga cct cgg gga cca aga        1584
   Trp Ala Lys Asp Cys Pro Lys Lys Pro Arg Gly Pro Arg Gly Pro Arg
           515                 520                 525
   ccc cag acc tcc ctc ctg acc cta gat gac tag gga ggt cag ggt cag        1632
   Pro Gln Thr Ser Leu Leu Thr Leu Asp Asp     Gly Gly Gln Gly Gln
       530                 535                     540
   gag ccc ccc cct gaa ccc agg ata acc ctc aaa gtc ggg ggg caa ccc        1680
   Glu Pro Pro Pro Glu Pro Arg Ile Thr Leu Lys Val Gly Gly Gln Pro
       545                 550                 555
   gtc acc ttc ctg gta gat act ggg gcc caa cac tcc gtg ctg acc caa        1728
   Val Thr Phe Leu Val Asp Thr Gly Ala Gln His Ser Val Leu Thr Gln
   560                 565                 570                 575
   aat cct gga ccc cta agt gat aag tct gcc tgg gtc caa ggg gct act        1776
   Asn Pro Gly Pro Leu Ser Asp Lys Ser Ala Trp Val Gln Gly Ala Thr
                   580                 585                 590
   gga gga aag cgg tat cgc tgg acc acg gat cgc aaa gta cat cta gct        1824
   Gly Gly Lys Arg Tyr Arg Trp Thr Thr Asp Arg Lys Val His Leu Ala
               595                 600                 605
   acc ggt aag gtc acc cac tct ttc ctc cat gta cca gac tgt ccc tat        1872
   Thr Gly Lys Val Thr His Ser Phe Leu His Val Pro Asp Cys Pro Tyr
           610                 615                 620
   cct ctg tta gga aga gat ttg ctg act aaa cta aaa gcc caa atc cac      1920
   Pro Leu Leu Gly Arg Asp Leu Leu Thr Lys Leu Lys Ala Gln Ile His
       625                 630                 635
   ttt gag gga tca gga gct cag gtt atg gga cca atg ggg cag ccc ctg      1968
   Phe Glu Gly Ser Gly Ala Gln Val Met Gly Pro Met Gly Gln Pro Leu
   640                 645                 650                 655
   caa gtg ttg acc cta aat ata gaa gat gag cat cgg cta cat gag acc      2016
   Gln Val Leu Thr Leu Asn Ile Glu Asp Glu His Arg Leu His Glu Thr
                   660                 665                 670
   tca aaa gag cca gat gtt tct cta ggg tcc aca tgg ctg tct gat ttt      2064
   Ser Lys Glu Pro Asp Val Ser Leu Gly Ser Thr Trp Leu Ser Asp Phe
               675                 680                 685
   cct cag gcc tgg gcg gaa acc ggg ggc atg gga ctg gca gtt cgc caa      2112
   Pro Gln Ala Trp Ala Glu Thr Gly Gly Met Gly Leu Ala Val Arg Gln
           690                 695                 700
   gct cct ctg atc ata cct ctg aaa gca acc tct acc ccc gtg tcc ata      2160
   Ala Pro Leu Ile Ile Pro Leu Lys Ala Thr Ser Thr Pro Val Ser Ile
       705                 710                 715
   aaa caa tac ccc atg tca caa gaa gcc aga ctg ggg atc aag ccc cac      2208
   Lys Gln Tyr Pro Met Ser Gln Glu Ala Arg Leu Gly Ile Lys Pro His
   720                 725                 730                 735
   ata cag aga ctg ttg gac cag gga ata ctg gta ccc tgc cag tcc ccc      2256
   Ile Gln Arg Leu Leu Asp Gln Gly Ile Leu Val Pro Cys Gln Ser Pro
                   740                 745                 750
   tgg aac acg ccc ctg cta ccc gtt aag aaa cca ggg act aat gat tat      2304
   Trp Asn Thr Pro Leu Leu Pro Val Lys Lys Pro Gly Thr Asn Asp Tyr
               755                 760                 765
   agg cct gtc cag gat ctg aga gaa gtc aac aag cgg gtg gaa gac atc      2352
   Arg Pro Val Gln Asp Leu Arg Glu Val Asn Lys Arg Val Glu Asp Ile
           770                 775                 780
   cac ccc acc gtg ccc aac cct tac aac ctc ttg agc ggg ctc cca ccg      2400
   His Pro Thr Val Pro Asn Pro Tyr Asn Leu Leu Ser Gly Leu Pro Pro
       785                 790                 795
   tcc cac cag tgg tac act gtg ctt gat tta aag gat gcc ttt ttc tgc      2448
   Ser His Gln Trp Tyr Thr Val Leu Asp Leu Lys Asp Ala Phe Phe Cys
   800                 805                 810                 815
   ctg aga ctc cac ccc acc agt cag cct ctc ttc gcc ttt gag tgg aga      2496
   Leu Arg Leu His Pro Thr Ser Gln Pro Leu Phe Ala Phe Glu Trp Arg
                   820                 825                 830
   gat cca gag atg gga atc tca gga caa ttg acc tgg acc aga ctc cca      2544
   Asp Pro Glu Met Gly Ile Ser Gly Gln Leu Thr Trp Thr Arg Leu Pro
               835                 840                 845
   cag ggt ttc aaa aac agt ccc acc ctg ttt gat gag gca ctg cac aga      2592
   Gln Gly Phe Lys Asn Ser Pro Thr Leu Phe Asp Glu Ala Leu His Arg
           850                 855                 860
   gac cta gca gac ttc cgg atc cag cac cca gac ttg atc ctg cta cag      2640
   Asp Leu Ala Asp Phe Arg Ile Gln His Pro Asp Leu Ile Leu Leu Gln
       865                 870                 875
   tac gtg gat gac tta ctg ctg gcc gcc act tct gag cta gac tgc caa       2688
   Tyr Val Asp Asp Leu Leu Leu Ala Ala Thr Ser Glu Leu Asp Cys Gln
   880                 885                 890                 895
   caa ggt act cgg gcc ctg tta caa acc cta ggg aac ctc ggg tat cgg       2736
   Gln Gly Thr Arg Ala Leu Leu Gln Thr Leu Gly Asn Leu Gly Tyr Arg
                   900                 905                 910
   gcc tcg gcc aag aaa gcc caa att tgc cag aaa cag gtc aag tat ctg       2784
   Ala Ser Ala Lys Lys Ala Gln Ile Cys Gln Lys Gln Val Lys Tyr Leu
               915                 920                 925
   ggg tat ctt cta aaa gag ggt cag aga tgg ctg act gag gcc aga aaa       2832
   Gly Tyr Leu Leu Lys Glu Gly Gln Arg Trp Leu Thr Glu Ala Arg Lys
           930                 935                 940
   gag act gtg atg ggg cag cct act ccg aag acc cct cga caa cta agg       2880
   Glu Thr Val Met Gly Gln Pro Thr Pro Lys Thr Pro Arg Gln Leu Arg
       945                 950                 955
   gag ttc cta ggg acg gca ggc ttc tgt cgc ctc tgg atc cct ggg ttt       2928
   Glu Phe Leu Gly Thr Ala Gly Phe Cys Arg Leu Trp Ile Pro Gly Phe
   960                 965                 970                 975
   gca gaa atg gca gcc ccc ttg tac cct ctc acc aaa acg ggg act ctg       2976
   Ala Glu Met Ala Ala Pro Leu Tyr Pro Leu Thr Lys Thr Gly Thr Leu
                   980                 985                 990
   ttt aat tgg ggc cca gac caa caa aag  gcc tat caa gaa atc  aag caa     3024
   Phe Asn Trp Gly Pro Asp Gln Gln Lys  Ala Tyr Gln Glu Ile  Lys Gln
               995                 1000                1005
   gct ctt cta  act gcc cca gcc ctg  ggg ttg cca gat ttg  act aag        3069
   Ala Leu Leu  Thr Ala Pro Ala Leu  Gly Leu Pro Asp Leu  Thr Lys
           1010                1015                1020
   ccc ttt gaa  ctc ttt gtc gac gag  aag cag ggc tac gcc  aaa ggt        3114
   Pro Phe Glu  Leu Phe Val Asp Glu  Lys Gln Gly Tyr Ala  Lys Gly
           1025                1030                1035
   gtc cta acg  caa aaa ctg gga cct  tgg cgt cgg ccg gtg  gcc tac        3159
   Val Leu Thr  Gln Lys Leu Gly Pro  Trp Arg Arg Pro Val  Ala Tyr
           1040                1045                1050
   ctg tcc aaa  aag cta gac cca gta  gca gct ggg tgg ccc  cct tgc        3204
   Leu Ser Lys  Lys Leu Asp Pro Val  Ala Ala Gly Trp Pro  Pro Cys
           1055                1060                1065
   cta cgg atg  gta gca gcc att gcc  gta ctg aca aag gat  gca ggc        3249
   Leu Arg Met  Val Ala Ala Ile Ala  Val Leu Thr Lys Asp  Ala Gly
           1070                1075                1080
   aag cta acc  atg gga cag cca cta  gtc att ctg gcc ccc  cat gca        3294
   Lys Leu Thr  Met Gly Gln Pro Leu  Val Ile Leu Ala Pro  His Ala
           1085                1090                1095
   gta gag gca  cta gtc aaa caa ccc  ccc gac cgc tgg ctt  tcc aat        3339
   Val Glu Ala  Leu Val Lys Gln Pro  Pro Asp Arg Trp Leu  Ser Asn
           1100                1105                1110
   ctg gcc ccc  cat gca gta gag gca  cta gtt aag caa ccc  ccc gat        3384
   Leu Ala Pro  His Ala Val Glu Ala  Leu Val Lys Gln Pro  Pro Asp
           1115                1120                1125
   cgc tgg ctc  tcc aac gcc cgg atg  act cac tat cag gcc  ttg ctt        3429
   Arg Trp Leu  Ser Asn Ala Arg Met  Thr His Tyr Gln Ala  Leu Leu
           1130                1135                1140
   ttg gac acg  gac cgg gtc cag ttc  gga ccg gtg gta gcc  ctg aac        3474
   Leu Asp Thr  Asp Arg Val Gln Phe  Gly Pro Val Val Ala  Leu Asn
           1145                1150                1155
   ccg gct acg  ctg ctc cca ctg cct  gag gaa ggg ctg caa  cac aac        3519
   Pro Ala Thr  Leu Leu Pro Leu Pro  Glu Glu Gly Leu Gln  His Asn
           1160                1165                1170
   tgc ctt gat  atc ctg gcc gaa gcc  cac gga acc cga ccc  gac cta        3564
   Cys Leu Asp  Ile Leu Ala Glu Ala  His Gly Thr Arg Pro  Asp Leu
           1175                1180                1185
   acg gac cag  ccg ctc cca gac gcc  gac cac acc tgg tac  acg gat        3609
   Thr Asp Gln  Pro Leu Pro Asp Ala  Asp His Thr Trp Tyr  Thr Asp
           1190                1195                1200
   gga agc agt  ctc tta caa gag gga  cag cgt aag gcg gga  gct gcg        3654
   Gly Ser Ser  Leu Leu Gln Glu Gly  Gln Arg Lys Ala Gly  Ala Ala
           1205                1210                1215
   gtg acc acc  gag acc gag gta atc  tgg gct aaa gcc ctg  cca gcc        3699
   Val Thr Thr  Glu Thr Glu Val Ile  Trp Ala Lys Ala Leu  Pro Ala
           1220                1225                1230
   ggg aca tcc  gct cag cgg gct gaa  ctg ata gca ctc acc  cag gcc        3744
   Gly Thr Ser  Ala Gln Arg Ala Glu  Leu Ile Ala Leu Thr  Gln Ala
           1235                1240                1245
   cta aag atg  gca gaa ggt aag aag  cta aat gtt tat act  gat agc        3789
   Leu Lys Met  Ala Glu Gly Lys Lys  Leu Asn Val Tyr Thr  Asp Ser
           1250                1255                1260
   cgt tat gct  ttt gct act gcc cat  atc cat gga gaa ata  tac aga        3834
   Arg Tyr Ala  Phe Ala Thr Ala His  Ile His Gly Glu Ile  Tyr Arg
           1265                1270                1275
   agg cgt ggg  ttg ctc aca tca gaa  ggc aaa gag atc aaa  aat aaa        3879
   Arg Arg Gly  Leu Leu Thr Ser Glu  Gly Lys Glu Ile Lys  Asn Lys
           1280                1285                1290
   gac gag atc  ttg gcc cta cta aaa  gcc ctc ttt ctg ccc  aaa aga        3924
   Asp Glu Ile  Leu Ala Leu Leu Lys  Ala Leu Phe Leu Pro  Lys Arg
           1295                1300                1305
   ctt agc ata  atc cat tgt cca gga  cat caa aag gga cac  agc gcc        3969
   Leu Ser Ile  Ile His Cys Pro Gly  His Gln Lys Gly His  Ser Ala
           1310                1315                1320
   gag gct aga  ggc aac cgg atg gct  gac caa gcg gcc cga  aag gca        4014
   Glu Ala Arg  Gly Asn Arg Met Ala  Asp Gln Ala Ala Arg  Lys Ala
           1325                1330                1335
   gcc atc aca  gag act cca gac acc  tct acc ctc ctc ata  gaa aat        4059
   Ala Ile Thr  Glu Thr Pro Asp Thr  Ser Thr Leu Leu Ile  Glu Asn
           1340                1345                1350
   tca tca ccc  tac acc tca gaa cat  ttt cat tac aca gtg  act gat        4104
   Ser Ser Pro Tyr Thr Ser Glu His Phe His Tyr Thr Val Thr Asp
           1355                1360                1365
   ata aag gac cta acc aag ttg ggg gcc att tat gat aaa aca aag        4149
   Ile Lys Asp Leu Thr Lys Leu Gly Ala Ile Tyr Asp Lys Thr Lys
           1370                1375                1380
   aag tat tgg gtc tac caa gga aaa cct gtg atg cct gac cag ttt        4194
   Lys Tyr Trp Val Tyr Gln Gly Lys Pro Val Met Pro Asp Gln Phe
           1385                1390                1395
   act ttt gaa tta tta gac ttt ctt cat cag ctg act cac ctc agc        4239
   Thr Phe Glu Leu Leu Asp Phe Leu His Gln Leu Thr His Leu Ser
           1400                1405                1410
   ttc tca aaa atg aag gct ctc cta gag aga agc cac agt ccc tac        4284
   Phe Ser Lys Met Lys Ala Leu Leu Glu Arg Ser His Ser Pro Tyr
           1415                1420                1425
   tac atg ctg aac cgg gat cga aca ctc aaa aat atc act gag acc        4329
   Tyr Met Leu Asn Arg Asp Arg Thr Leu Lys Asn Ile Thr Glu Thr
           1430                1435                1440
   tgc aaa gct tgt gca caa gtc aac gcc agc aag tct gcc gtt aaa        4374
   Cys Lys Ala Cys Ala Gln Val Asn Ala Ser Lys Ser Ala Val Lys
           1445                1450                1455
   cag gga act agg gtc cgc ggg cat cgg ccc ggc act cat tgg gag        4419
   Gln Gly Thr Arg Val Arg Gly His Arg Pro Gly Thr His Trp Glu
           1460                1465                1470
   atc gat ttc acc gag ata aag ccc gga ttg tat ggc tat aaa tat        4464
   Ile Asp Phe Thr Glu Ile Lys Pro Gly Leu Tyr Gly Tyr Lys Tyr
           1475                1480                1485
   ctt cta gtt ttt ata gat acc ttt tct ggc tgg ata gaa gcc ttc        4509
   Leu Leu Val Phe Ile Asp Thr Phe Ser Gly Trp Ile Glu Ala Phe
           1490                1495                1500
   cca acc aag aaa gaa acc gcc aag gtc gta acc aag aag cta cta        4554
   Pro Thr Lys Lys Glu Thr Ala Lys Val Val Thr Lys Lys Leu Leu
           1505                1510                1515
   gag gag atc ttc ccc agg ttc ggc atg cct cag gta ttg gga act        4599
   Glu Glu Ile Phe Pro Arg Phe Gly Met Pro Gln Val Leu Gly Thr
           1520                1525                1530
   gac aat ggg cct gcc ttc gtc tcc aag gtg agt cag aca gtg gcc        4644
   Asp Asn Gly Pro Ala Phe Val Ser Lys Val Ser Gln Thr Val Ala
           1535                1540                1545
   gat ctg ttg ggg att gat tgg aaa tta cat tgt gca tac aga ccc        4689
   Asp Leu Leu Gly Ile Asp Trp Lys Leu His Cys Ala Tyr Arg Pro
           1550                1555                1560
   caa agc tca ggc cag gta gaa aga atg aat aga acc atc aag gag        4734
   Gln Ser Ser Gly Gln Val Glu Arg Met Asn Arg Thr Ile Lys Glu
           1565                1570                1575
   act tta act aaa tta acg ctt gca act ggc tct aga gac tgg gtg        4779
   Thr Leu Thr Lys Leu Thr Leu Ala Thr Gly Ser Arg Asp Trp Val
           1580                1585                1590
   ctc cta ctc  ccc tta gcc ctg tac  cga gcc cgc aac acg  ccg ggc        4824
   Leu Leu Leu  Pro Leu Ala Leu Tyr  Arg Ala Arg Asn Thr  Pro Gly
           1595                 1600                 1605
   ccc cat ggc  ctc acc cca tat gag  atc tta tat ggg gca  ccc ccg        4869
   Pro His Gly  Leu Thr Pro Tyr Glu  Ile Leu Tyr Gly Ala  Pro Pro
           1610                 1615                 1620
   ccc ctt gta  aac ttc cct gac cct  gac atg aca aga gtt  act aac        4914
   Pro Leu Val  Asn Phe Pro Asp Pro  Asp Met Thr Arg Val  Thr Asn
           1625                 1630                 1635
   agc ccc tct  ctc caa gct cac tta  cag gct ctc tac tta  gtc cag        4959
   Ser Pro Ser  Leu Gln Ala His Leu  Gln Ala Leu Tyr Leu  Val Gln
           1640                 1645                 1650
   cac gaa gtc  tgg aga cct ctg gcg  gca gcc tac caa gaa  caa ctg        5004
   His Glu Val  Trp Arg Pro Leu Ala  Ala Ala Tyr Gln Glu  Gln Leu
           1655                 1660                 1665
   gac cga ccg  gtg gta cct cac cct  tac cga gtc ggc gac  aca gtg        5049
   Asp Arg Pro  Val Val Pro His Pro  Tyr Arg Val Gly Asp  Thr Val
           1670                 1675                 1680
   tgg gtc cgc  cga cac cag act aag  aac cta gaa cct cgc  tgg aaa        5094
   Trp Val Arg  Arg His Gln Thr Lys  Asn Leu Glu Pro Arg  Trp Lys
           1685                 1690                 1695
   gga cct tac  aca gtc ctg ctg acc  acc ccc acc gcc ctc  aaa gta        5139
   Gly Pro Tyr  Thr Val Leu Leu Thr  Thr Pro Thr Ala Leu  Lys Val
           1700                 1705                 1710
   gac ggc atc  gca gct tgg ata cac  gcc gcc cac gtg aag  gct gcc        5184
   Asp Gly Ile  Ala Ala Trp Ile His  Ala Ala His Val Lys  Ala Ala
           1715                 1720                 1725
   gac ccc ggg  ggt gga cca tcc tct  aga ctg aca tgg cgc  gtt caa        5229
   Asp Pro Gly  Gly Gly Pro Ser Ser  Arg Leu Thr Trp Arg  Val Gln
           1730                 1735                 1740
   cgc tct caa  aac ccc tta aaa ata  agg tta acc cgc gag  gcc ccc        5274
   Arg Ser Gln  Asn Pro Leu Lys Ile  Arg Leu Thr Arg Glu  Ala Pro
           1745                 1750                 1755
   taa                                                                   5277
<210> 2
   <211> 538
   <212> PRT
   <213> Murine leukemia virus
<400> 2
   Met Gly Gln Thr Val Thr Thr Pro Leu Ser Leu Thr Leu Gly His Trp
   1               5                   10                  15
   Lys Asp Val Glu Arg Ile Ala His Asn Gln Ser Val Asp Val Lys Lys
               20                  25                  30
   Arg Arg Trp Val Thr Phe Cys Ser Ala Glu Trp Pro Thr Phe Asn Val
               35                  40                  45
   Gly Trp Pro Arg Asp Gly Thr Phe Asn Arg Asp Leu Ile Thr Gln Val
       50                  55                  60
   Lys Ile Lys Val Phe Ser Pro Gly Pro His Gly His Pro Asp Gln Val
   65                  70                  75                  80
   Pro Tyr Ile Val Thr Trp Glu Ala Leu Ala Phe Asp Pro Pro Pro Trp
                   85                  90                  95
   Val Lys Pro Phe Val His Pro Lys Pro Pro Pro Pro Leu Pro Pro Ser
               100                 105                 110
   Ala Pro Ser Leu Pro Leu Glu Pro Pro Arg Ser Thr Pro Pro Arg Ser
           115                 120                 125
   Ser Leu Tyr Pro Ala Leu Thr Pro Ser Leu Gly Ala Lys Pro Lys Pro
       130                 135                 140
   Gln Val Leu Ser Asp Ser Gly Gly Pro Leu Ile Asp Leu Leu Thr Glu
   145                 150                 155                 160
   Asp Pro Pro Pro Tyr Arg Asp Pro Arg Pro Pro Pro Ser Asp Arg Asp
                   165                 170                 175
   Gly Asn Gly Gly Glu Ala Thr Pro Ala Gly Glu Ala Pro Asp Pro Ser
               180                 185                 190
   Pro Met Ala Ser Arg Leu Arg Gly Arg Arg Glu Pro Pro Val Ala Asp
           195                 200                 205
   Ser Thr Thr Ser Gln Ala Phe Pro Leu Arg Ala Gly Gly Asn Gly Gln
       210                 215                 220
   Leu Gln Tyr Trp Pro Phe Ser Ser Ser Asp Leu Tyr Asn Trp Lys Asn
   225                 230                 235                 240
   Asn Asn Pro Ser Phe Ser Glu Asp Pro Gly Lys Leu Thr Ala Leu Ile
                   245                 250                 255
   Glu Ser Val Leu Ile Thr His Gln Pro Thr Trp Asp Asp Cys Gln Gln
               260                 265                 270
   Leu Leu Gly Thr Leu Leu Thr Gly Glu Glu Lys Gln Arg Val Leu Leu
           275                 280                 285
   Glu Ala Arg Lys Ala Val Arg Gly Asp Asp Gly Arg Pro Thr Gln Leu
       290                 295                 300
   Pro Asn Glu Val Asp Ala Ala Phe Pro Leu Glu Arg Pro Asp Trp Asp
   305                 310                 315                 320
   Tyr Thr Thr Gln Ala Gly Arg Asn His Leu Val His Tyr Arg Gln Leu
                   325                 330                 335
   Leu Leu Ala Gly Leu Gln Asn Ala Gly Arg Ser Pro Thr Asn Leu Ala
               340                 345                 350
   Lys Val Lys Gly Ile Thr Gln Gly Pro Asn Glu Ser Pro Ser Ala Phe
           355                 360                 365
   Leu Glu Arg Leu Lys Glu Ala Tyr Arg Arg Tyr Thr Pro Tyr Asp Pro
       370                 375                 380
   Glu Asp Pro Gly Gln Glu Thr Asn Val Ser Met Ser Phe Ile Trp Gln
   385                 390                 395                 400
   Ser Ala Pro Asp Ile Gly Arg Lys Leu Glu Arg Leu Glu Asp Leu Lys
                   405                 410                 415
   Asn Lys Thr Leu Gly Asp Leu Val Arg Glu Ala Glu Lys Ile Phe Asn
               420                 425                 430
   Lys Arg Glu Thr Pro Glu Glu Arg Glu Glu Arg Ile Arg Arg Glu Thr
           435                 440                 445
   Glu Glu Lys Glu Glu Arg Arg Arg Thr Glu Asp Glu Gln Lys Glu Lys
       450                 455                 460
   Glu Arg Asp Arg Arg Arg His Arg Glu Met Ser Lys Leu Leu Ala Thr
   465                 470                 475                 480
   Val Val Ser Gly Gln Lys Gln Asp Arg Gln Gly Gly Glu Arg Arg Arg
                   485                 490                 495
   Ser Gln Leu Asp Arg Asp Gln Cys Ala Tyr Cys Lys Glu Lys Gly His
               500                 505                 510
   Trp Ala Lys Asp Cys Pro Lys Lys Pro Arg Gly Pro Arg Gly Pro Arg
           515                 520                 525
   Pro Gln Thr Ser Leu Leu Thr Leu Asp Asp
       530                 535
<210> 3
   <211> 1219
   <212> PRT
   <213> Murine leukemia virus
<400> 3
   Gly Gly Gln Gly Gln Glu Pro Pro Pro Glu Pro Arg Ile Thr Leu Lys
   1               5                   10                  15
   Val Gly Gly Gln Pro Val Thr Phe Leu Val Asp Thr Gly Ala Gln His
               20                  25                  30
   Ser Val Leu Thr Gln Asn Pro Gly Pro Leu Ser Asp Lys Ser Ala Trp
           35                  40                  45
   Val Gln Gly Ala Thr Gly Gly Lys Arg Tyr Arg Trp Thr Thr Asp Arg
       50                  55                  60
   Lys Val His Leu Ala Thr Gly Lys Val Thr His Ser Phe Leu His Val
   65                  70                  75                  80
   Pro Asp Cys Pro Tyr Pro Leu Leu Gly Arg Asp Leu Leu Thr Lys Leu
                   85                  90                  95
   Lys Ala Gln Ile His Phe Glu Gly Ser Gly Ala Gln Val Met Gly Pro
               100                 105                 110
   Met Gly Gln Pro Leu Gln Val Leu Thr Leu Asn Ile Glu Asp Glu His
           115                 120                 125
   Arg Leu His Glu Thr Ser Lys Glu Pro Asp Val Ser Leu Gly Ser Thr
       130                 135                 140
   Trp Leu Ser Asp Phe Pro Gln Ala Trp Ala Glu Thr Gly Gly Met Gly
   145                 150                 155                 160
   Leu Ala Val Arg Gln Ala Pro Leu Ile Ile Pro Leu Lys Ala Thr Ser
                   165                 170                 175
   Thr Pro Val Ser Ile Lys Gln Tyr Pro Met Ser Gln Glu Ala Arg Leu
               180                 185                 190
   Gly Ile Lys Pro His Ile Gln Arg Leu Leu Asp Gln Gly Ile Leu Val
           195                 200                 205
   Pro Cys Gln Ser Pro Trp Asn Thr Pro Leu Leu Pro Val Lys Lys Pro
       210                 215                 220
   Gly Thr Asn Asp Tyr Arg Pro Val Gln Asp Leu Arg Glu Val Asn Lys
   225 230 235 240
   Arg Val Glu Asp Ile His Pro Thr Val Pro Asn Pro Tyr Asn Leu Leu
   245 250 255
   Ser Gly Leu Pro Pro Ser His Gln Trp Tyr Thr Val Leu Asp Leu Lys
   260 265 270
   Asp Ala Phe Phe Cys Leu Arg Leu His Pro Thr Ser Gln Pro Leu Phe
   275 280 285
   Ala Phe Glu Trp Arg Asp Pro Glu Met Gly Ile Ser Gly Gln Leu Thr
   290 295 300
   Trp Thr Arg Leu Pro Gln Gly Phe Lys Asn Ser Pro Thr Leu Phe Asp
   305 310 315 320
   Glu Ala Leu His Arg Asp Leu Ala Asp Phe Arg Ile Gln His Pro Asp
   325 330 335
   Leu Ile Leu Leu Gln Tyr Val Asp Asp Leu Leu Leu Ala Ala Thr Ser
   340 345 350
   Glu Leu Asp Cys Gln Gln Gly Thr Arg Ala Leu Leu Gln Thr Leu Gly
   355 360 365
   Asn Leu Gly Tyr Arg Ala Ser Ala Lys Lys Ala Gln Ile Cys Gln Lys
   370 375 380
   Gln Val Lys Tyr Leu Gly Tyr Leu Leu Lys Glu Gly Gln Arg Trp Leu
   385 390 395 400
   Thr Glu Ala Arg Lys Glu Thr Val Met Gly Gln Pro Thr Pro Lys Thr
   405 410 415
   Pro Arg Gln Leu Arg Glu Phe Leu Gly Thr Ala Gly Phe Cys Arg Leu
   420 425 430
   Trp Ile Pro Gly Phe Ala Glu Met Ala Ala Pro Leu Tyr Pro Leu Thr
   435 440 445
   Lys Thr Gly Thr Leu Phe Asn Trp Gly Pro Asp Gln Gln Lys Ala Tyr
   450 455 460
   Gln Glu Ile Lys Gln Ala Leu Leu Thr Ala Pro Ala Leu Gly Leu Pro
   465 470 475 480
   Asp Leu Thr Lys Pro Phe Glu Leu Phe Val Asp Glu Lys Gln Gly Tyr
   485 490 495
   Ala Lys Gly Val Leu Thr Gln Lys Leu Gly Pro Trp Arg Arg Pro Val
   500 505 510
   Ala Tyr Leu Ser Lys Lys Leu Asp Pro Val Ala Ala Gly Trp Pro Pro
   515 520 525
   Cys Leu Arg Met Val Ala Ala Ile Ala Val Leu Thr Lys Asp Ala Gly
   530 535 540
   Lys Leu Thr Met Gly Gln Pro Leu Val Ile Leu Ala Pro His Ala Val
   545 550 555 560
   Glu Ala Leu Val Lys Gln Pro Pro Asp Arg Trp Leu Ser Asn Leu Ala
   565 570 575
   Pro His Ala Val Glu Ala Leu Val Lys Gln Pro Pro Asp Arg Trp Leu
   580 585 590
   Ser Asn Ala Arg Met Thr His Tyr Gln Ala Leu Leu Leu Asp Thr Asp
   595 600 605
   Arg Val Gln Phe Gly Pro Val Val Ala Leu Asn Pro Ala Thr Leu Leu
   610 615 620
   Pro Leu Pro Glu Glu Gly Leu Gln His Asn Cys Leu Asp Ile Leu Ala
   625 630 635 640
   Glu Ala His Gly Thr Arg Pro Asp Leu Thr Asp Gln Pro Leu Pro Asp
   645 650 655
   Ala Asp His Thr Trp Tyr Thr Asp Gly Ser Ser Leu Leu Gln Glu Gly
   660 665 670
   Gln Arg Lys Ala Gly Ala Ala Val Thr Thr Glu Thr Glu Val Ile Trp
   675 680 685
   Ala Lys Ala Leu Pro Ala Gly Thr Ser Ala Gln Arg Ala Glu Leu Ile
   690 695 700
   Ala Leu Thr Gln Ala Leu Lys Met Ala Glu Gly Lys Lys Leu Asn Val
   705 710 715 720
   Tyr Thr Asp Ser Arg Tyr Ala Phe Ala Thr Ala His Ile His Gly Glu
                   725                 730                 735
   Ile Tyr Arg Arg Arg Gly Leu Leu Thr Ser Glu Gly Lys Glu Ile Lys
               740                 745                 750
   Asn Lys Asp Glu Ile Leu Ala Leu Leu Lys Ala Leu Phe Leu Pro Lys
           755                 760                 765
   Arg Leu Ser Ile Ile His Cys Pro Gly His Gln Lys Gly His Ser Ala
       770                 775                 780
   Glu Ala Arg Gly Asn Arg Met Ala Asp Gln Ala Ala Arg Lys Ala Ala
   785                 790                 795                 800
   Ile Thr Glu Thr Pro Asp Thr Ser Thr Leu Leu Ile Glu Asn Ser Ser
                   805                 810                 815
   Pro Tyr Thr Ser Glu His Phe His Tyr Thr Val Thr Asp Ile Lys Asp
               820                 825                 830
   Leu Thr Lys Leu Gly Ala Ile Tyr Asp Lys Thr Lys Lys Tyr Trp Val
           835                 840                 845
   Tyr Gln Gly Lys Pro Val Met Pro Asp Gln Phe Thr Phe Glu Leu Leu
       850                 855                 860
   Asp Phe Leu His Gln Leu Thr His Leu Ser Phe Ser Lys Met Lys Ala
   865                 870                 875                 880
   Leu Leu Glu Arg Ser His Ser Pro Tyr Tyr Met Leu Asn Arg Asp Arg
                   885                 890                 895
   Thr Leu Lys Asn Ile Thr Glu Thr Cys Lys Ala Cys Ala Gln Val Asn
               900                 905                 910
   Ala Ser Lys Ser Ala Val Lys Gln Gly Thr Arg Val Arg Gly His Arg
           915                 920                 925
   Pro Gly Thr His Trp Glu Ile Asp Phe Thr Glu Ile Lys Pro Gly Leu
       930                 935                 940
   Tyr Gly Tyr Lys Tyr Leu Leu Val Phe Ile Asp Thr Phe Ser Gly Trp
   945                 950                 955                 960
   Ile Glu Ala Phe Pro Thr Lys Lys Glu Thr Ala Lys Val Val Thr Lys
                   965                 970                 975
   Lys Leu Leu Glu Glu Ile Phe Pro Arg Phe Gly Met Pro Gln Val Leu
   980 985 990
   Gly Thr Asp Asn Gly Pro Ala Phe Val Ser Lys Val Ser Gln Thr Val
   995 1000 1005
   Ala Asp Leu Leu Gly Ile Asp Trp Lys Leu His Cys Ala Tyr Arg
   1010 1015 1020
   Pro Gln Ser Ser Gly Gln Val Glu Arg Met Asn Arg Thr Ile Lys
   1025 1030 1035
   Glu Thr Leu Thr Lys Leu Thr Leu Ala Thr Gly Ser Arg Asp Trp
   1040 1045 1050
   Val Leu Leu Leu Pro Leu Ala Leu Tyr Arg Ala Arg Asn Thr Pro
   1055 1060 1065
   Gly Pro His Gly Leu Thr Pro Tyr Glu Ile Leu Tyr Gly Ala Pro
   1070 1075 1080
   Pro Pro Leu Val Asn Phe Pro Asp Pro Asp Met Thr Arg Val Thr
   1085 1090 1095
   Asn Ser Pro Ser Leu Gln Ala His Leu Gln Ala Leu Tyr Leu Val
   1100 1105 1110
   Gln His Glu Val Trp Arg Pro Leu Ala Ala Ala Tyr Gln Glu Gln
   1115 1120 1125
   Leu Asp Arg Pro Val Val Pro His Pro Tyr Arg Val Gly Asp Thr
   1130 1135 1140
   Val Trp Val Arg Arg His Gln Thr Lys Asn Leu Glu Pro Arg Trp
   1145 1150 1155
   Lys Gly Pro Tyr Thr Val Leu Leu Thr Thr Pro Thr Ala Leu Lys
   1160 1165 1170
   Val Asp Gly Ile Ala Ala Trp Ile His Ala Ala His Val Lys Ala
   1175 1180 1185
   Ala Asp Pro Gly Gly Gly Pro Ser Ser Arg Leu Thr Trp Arg Val
   1190 1195 1200
   Gln Arg Ser Gln Asn Pro Leu Lys Ile Arg Leu Thr Arg Glu Ala
   1205 1210 1215
   Pro
<210> 4
   <211> 721
   <212> PRT
   <213> Artificial Sequence
<220>
   <223> MLV protein fragment
<400> 4
   Gly Gly Ala Gly Ala Cys Gly Thr Cys Cys Cys Ala Gly Gly Gly Ala
   1               5                   10                  15
   Cys Thr Thr Cys Gly Gly Gly Gly Gly Cys Cys Gly Thr Thr Thr Thr
               20                  25                  30
   Thr Gly Thr Gly Gly Cys Cys Cys Gly Ala Cys Cys Thr Gly Ala Gly
           35                  40                  45
   Thr Cys Cys Ala Ala Ala Ala Ala Thr Cys Cys Cys Gly Ala Thr Cys
       50                  55                  60
   Gly Thr Thr Thr Thr Gly Gly Ala Cys Thr Cys Thr Thr Thr Gly Gly
   65                  70                  75                  80
   Thr Gly Cys Ala Cys Cys Cys Cys Cys Cys Thr Thr Ala Gly Ala Gly
                   85                  90                  95
   Gly Ala Gly Gly Gly Ala Thr Ala Thr Gly Thr Gly Gly Thr Thr Cys
               100                 105                 110
   Thr Gly Gly Thr Ala Gly Gly Ala Gly Ala Cys Gly Ala Gly Ala Ala
           115                 120                 125
   Cys Cys Thr Ala Ala Ala Ala Cys Ala Gly Thr Thr Cys Cys Cys Gly
       130                 135                 140
   Cys Cys Thr Cys Cys Gly Thr Cys Thr Gly Ala Ala Thr Thr Thr Thr
   145                 150                 155                 160
   Thr Gly Cys Thr Thr Thr Cys Gly Gly Thr Thr Thr Gly Gly Gly Ala
                   165                 170                 175
   Cys Cys Gly Ala Ala Gly Cys Cys Gly Cys Gly Cys Cys Gly Cys Gly
               180                 185                 190
   Cys Gly Thr Cys Thr Thr Gly Thr Cys Thr Gly Cys Thr Gly Cys Ala
           195                 200                 205
   Gly Cys Ala Thr Cys Gly Thr Thr Cys Thr Gly Thr Gly Thr Thr Gly
       210                 215                 220
   Thr Cys Thr Cys Thr Gly Thr Cys Thr Gly Ala Cys Thr Gly Thr Gly
   225                 230                 235                 240
   Thr Thr Thr Cys Thr Gly Thr Ala Thr Thr Thr Gly Thr Cys Thr Gly
                   245                 250                 255
   Ala Ala Ala Ala Thr Ala Thr Gly Gly Gly Cys Cys Ala Gly Ala Cys
               260                 265                 270
   Thr Gly Thr Thr Ala Cys Cys Ala Cys Thr Cys Cys Cys Thr Thr Ala
           275                 280                 285
   Ala Gly Thr Thr Thr Gly Ala Cys Cys Thr Thr Ala Gly Gly Thr Cys
       290                 295                 300
   Ala Cys Thr Gly Gly Ala Ala Ala Gly Ala Thr Gly Thr Cys Gly Ala
   305                 310                 315                 320
   Gly Cys Gly Gly Ala Thr Cys Gly Cys Thr Cys Ala Cys Ala Ala Cys
                   325                 330                 335
   Cys Ala Gly Thr Cys Gly Gly Thr Ala Gly Ala Thr Gly Thr Cys Ala
               340                 345                 350
   Ala Gly Ala Ala Gly Ala Gly Ala Cys Gly Thr Thr Gly Gly Gly Thr
           355                 360                 365
   Thr Ala Cys Cys Thr Thr Cys Thr Gly Cys Thr Cys Thr Gly Cys Ala
       370                 375                 380
   Gly Ala Ala Thr Gly Gly Cys Cys Ala Ala Cys Cys Thr Thr Thr Ala
   385                 390                 395                 400
   Ala Cys Gly Thr Cys Gly Gly Ala Thr Gly Gly Cys Cys Gly Cys Gly
                   405                 410                 415
   Ala Gly Ala Cys Gly Gly Cys Ala Cys Cys Thr Thr Thr Ala Ala Cys
               420                 425                 430
   Cys Gly Ala Gly Ala Cys Cys Thr Cys Ala Thr Cys Ala Cys Cys Cys
           435                 440                 445
   Ala Gly Gly Thr Thr Ala Ala Gly Ala Thr Cys Ala Ala Gly Gly Thr
       450                 455                 460
   Cys Thr Thr Thr Thr Cys Ala Cys Cys Thr Gly Gly Cys Cys Cys Gly
   465                 470                 475                 480
   Cys Ala Thr Gly Gly Ala Cys Ala Cys Cys Cys Ala Gly Ala Cys Cys
                   485                 490                 495
   Ala Gly Gly Thr Cys Cys Cys Cys Thr Ala Cys Ala Thr Cys Gly Thr
               500                 505                 510
   Gly Ala Cys Cys Thr Gly Gly Gly Ala Ala Gly Cys Cys Thr Thr Gly
           515                 520                 525
   Gly Cys Thr Thr Thr Thr Gly Ala Cys Cys Cys Cys Cys Cys Thr Cys
       530                 535                 540
   Cys Cys Thr Gly Gly Gly Thr Cys Ala Ala Gly Cys Cys Cys Thr Thr
   545                 550                 555                 560
   Thr Gly Thr Ala Cys Ala Cys Cys Cys Thr Ala Ala Gly Cys Cys Thr
                   565                 570                 575
   Cys Cys Gly Cys Cys Thr Cys Cys Thr Cys Thr Thr Cys Cys Thr Cys
               580                 585                 590
   Cys Ala Thr Cys Cys Gly Cys Cys Cys Cys Gly Thr Cys Thr Cys Thr
           595                 600                 605
   Cys Cys Cys Cys Cys Thr Thr Gly Ala Ala Cys Cys Thr Cys Cys Thr
       610                 615                 620
   Cys Gly Thr Thr Cys Gly Ala Cys Cys Cys Cys Gly Cys Cys Thr Cys
   625                 630                 635                 640
   Gly Ala Thr Cys Cys Thr Cys Cys Cys Thr Thr Thr Ala Thr Cys Cys
                   645                 650                 655
   Ala Gly Cys Cys Cys Thr Cys Ala Cys Thr Cys Cys Thr Thr Cys Thr
               660                 665                 670
   Cys Thr Ala Gly Gly Cys Gly Cys Cys Ala Ala Ala Cys Cys Thr Ala
           675                 680                 685
   Ala Ala Cys Cys Thr Cys Ala Ala Gly Thr Thr Cys Thr Thr Thr Cys
       690                 695                 700
   Thr Gly Ala Cys Ala Gly Thr Gly Gly Gly Gly Gly Gly Cys Cys Gly
   705                 710                 715                 720
   Cys

## Description

### TECHNICAL FIELD

The disclosure provides for retroviral replicating vectors (RRVs) and the identification of one or more immunosuppressive components that blockade Type I IFN production by RRVs and/or reduce tumor cell responses to IFN which contribute to tumor selectivity by RRVs.

### BACKGROUND

Type I interferon appears to contribute to the development of autoimmunity and disease progression in multiple autoimmune diseases including systemic lupus erythematosus (SLE), rheumatoid arthritis (RA), and multiple sclerosis (MS). In particular, patients with SLE often have high level of type I interferon (IFN) production in their blood and display an increased expression of type I IFN-induced gene expression profile. The reason for the continuous IFN-α production in these patients appears to be caused by the continuous activation of the plasmacytoid dendritic cells (pDCs) mediated by immune complexes such as autoantibodies via TLR7 and TLR9. In addition, the clinical observation that IFN-α treatment can induces autoimmunity suggests that type I IFN signalling plays a contributing role in development of autoimmune diseases.

Retroviral replicating vectors (RRVs) appear to be relatively non-inflammatory, weak immunogens, and, in rodent tumor models, replicate in target tumors without extensive replication elsewhere in healthy tissues.

### SUMMARY

The disclosure provides an immunosuppressive composition comprsing a non-enveloped, non-replicating viral-like particle (VLP) comprising a naked viral particle consisting of (i) structural gag and pol polypeptides from a gammaretrovirus; and/or (ii) a gamma retroviral gag-pol polyprotein, wherein the VLP lacks a viral genome, a lipid envelope, and envelope proteins. In an embodiment, the VLP comprises one or more immunosuppressive components associated with a gammaretrovirus that block Type I IFN productionIn a further embodiment, the immunosuppressive components block the activation of endosomal Toll-like receptors (TLRs) in pDCs and subsequent induction of Type I IFN production. In yet another embodiment of any of the foregoing the disclosure provides a composition coprising a VLP of any and further comprising an additional immunosuppressive agent. In yet another embodiment, the additional immunosuppressive agent is an anti-IFNα antibody or fragment thereof. In still another embodiment, the additional immunosuppressive agent is a steroid. In yet another embodiment, the composition is for use in the the treatment of an immune disorder.

The disclosure also provides a method of treating an immune disorder comprising administering to a subject a composition of any of the foregoing embodiment, wherein IFNα activity and/or production in inhibited. In one embodiment, the immune disorder is selected from the group consisting of rheumatoid arthritis, systemic lupus erythematosus, scleroderma, polymyositis, dermatomyositis, spondyloarthropathies such as ankylosing spondylitis, and Sjogren's syndrome. In another embodiment, the immune disorder is systemic lupus erythematosus.

The disclosure provides for the identification of one or more immunosuppressive components associated with retroviral replicating vectors (RRVs) (*e.g*., Toca 511) that contribute to RRV tumor specificity and efficacy.

In a particular embodiment, the disclosure provides that one or more immunosuppressive components disclosed herein blockade Type I IFN production by the RRVs. The one or more immunosuppressive components of the disclosure contribute to RRVs not inducing Type I IFN responses in cultured primary endothelial or non-transformed fibroblast cells, to RRVs not inducing Type I IFN responses in cultured tumor cells *in vitro,* and/or to RRVs not inducing IFNα upon incubation with plasmacytoid dendritic cells (pDCs). The one or more immunosuppressive components disclosed herein attenuates the inhibition of the RRV replication in tumor cells and/or attenuates the induction of IFN-regulated genes in response to an infection by the RRV. In another embodiment, the one or more immunosuppressive components disclosed herein block the activation of endosomal Toll-like receptors (TLRs) in pDCs and subsequent induction of Type I IFN production.

The disclosure provides that one or more immunosuppressive components disclosed herein contribute to tumor specificity and efficacy of the RRV. One or more immunosuppressive components of the disclosure are associated with an RRV.

The disclosure provides that one or more immunosuppressive components disclosed herein can be heat-inactivated, such as by heating at 56 °C for 30 minutes, so that one or more compononents are no longer immunosuppressive.

The disclosure provides that one or more immunosuppressive components disclosed herein comprise one or more of the following: proteins, lipids, carbohydrates, and nucleic acids. The one or more immunosuppressive components of the disclosure comprise proteins. The one or more immunosuppressive components of the disclosure comprise purified gag protein particles from a gamma retrovirus. The gamma retrovirus gag protein particles are derived form MLV or GAlV.

The disclosure provides that one or more immunosuppressive components disclosed herein facilitate tumor infection *in vivo* by an RRV.

### DESCRIPTION OF DRAWINGS

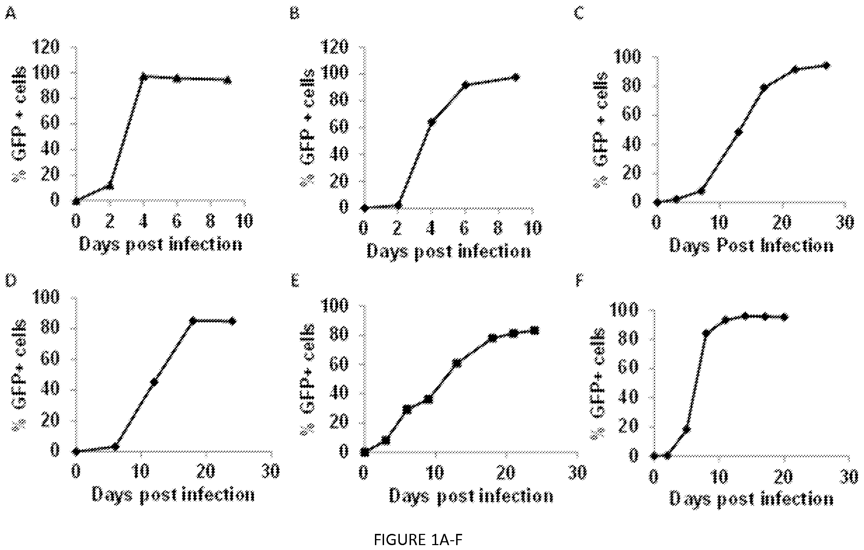

**Figure 1A-F** provides the replication kinetics of RRV-GFP in a panel of human tumor cell lines and non-transformed primary cells. **(A)** HT-1080, **(B)** U87, **(C)** LN-CaP, **(D)** Sup-T1 **(E)** WI-38 and **(F)** HUVEC. For each cell line, cells were infected with RRV-GFP at MOI of 0.1 at day 0 and passaged twice weekly. The replication kinetics of each vector was obtained by plotting the percentage of GFP-positive cells vs time.

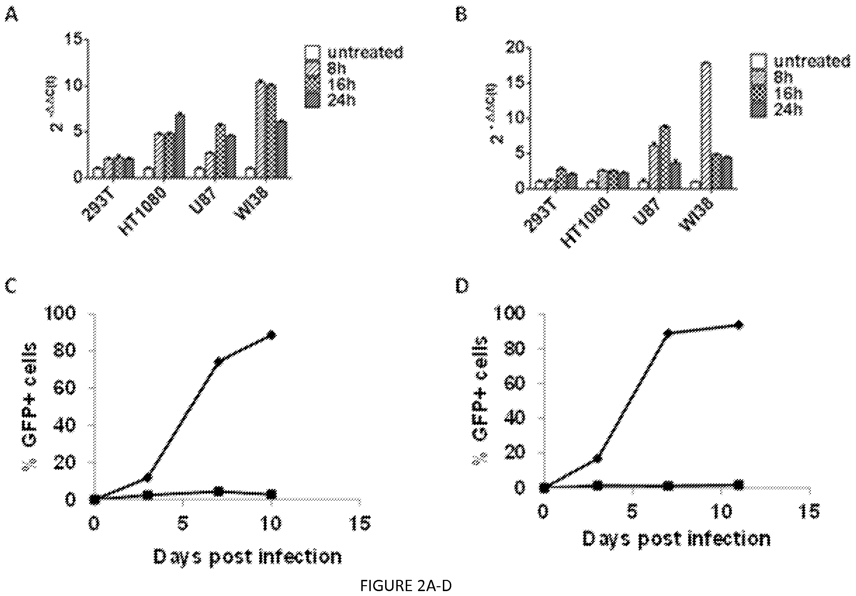

**Figure 2A-D** presents data indicating that U87-MG cells are responsive to Type I IFN signaling. Induction of Type I IFN-inducible gene **(A)** PKR and **(B)** APOBEC3G in a panel of established cell lines were induced by exogenous IFNα at 200 U/mL. Induction of PKR and APOBEC3G was measured at 8, 16 and 24 hours post addition of IFNα by qRT-PCR. Fold induction was calculated relative to untreated cells, and relative expression of each gene is expressed as 2^{-ΔΔ(Ct)}. The data shown represent mean ± SD, (n=3). **(C-D)** Replication kinetics of RRV-GFP in U87-MG cells in the presence and absence of **(C)** IFNα and **(D)** IFNβ. Diamond: untreated, square: treated with IFNα or IFNβ.

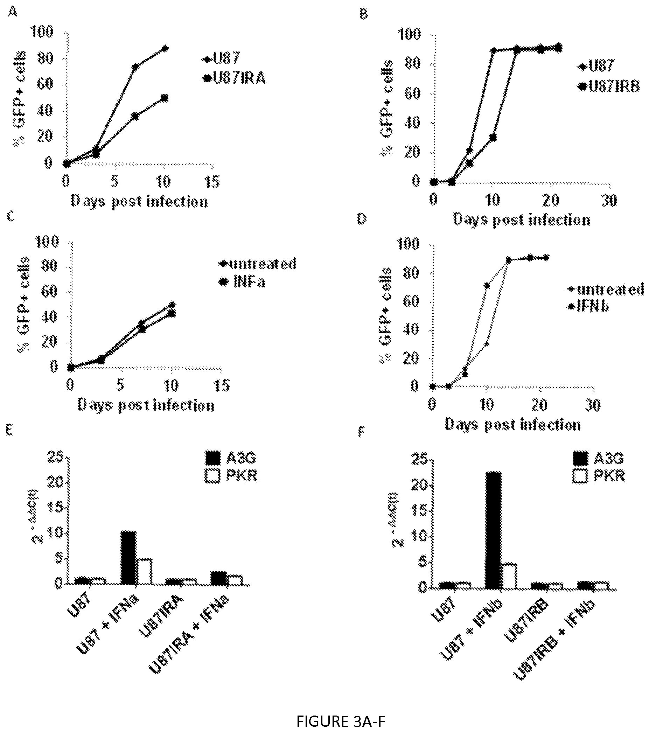

**Figure 3A-F** provides replication kinetics of RRV-GFP and induction of Type I IFN signaling in IFNα- and INFβ-resistant U87-MG cells. Replication kinetics of RRV-GFP in **(A)** IFNα-resistant (U87IRA) and **(B)** IFNβ-resistant (U87IRB) in the absence of IFNα/β. Replication kinetics of RRV-GFP in **(C)** U87IRA and **(D)** U87IRB cells in the absence and presence of IFNα/β. Cells were infected with RRV-GFP at MOI of 0.1 at day 0 and passaged twice weekly. **(E-F)** Induction of Type I IFN-inducible gene, PKR and APOBEC3G, in U87-MG parental, U87IRA and U87IRB cells. Cellular mRNA was extracted at 16 hours post addition of exogenous IFNα or IFNβ and expression levels were measured by qRT-PCR. Fold induction was calculated relative to untreated cells, and relative expression of each gene is expressed as 2^{-ΔΔ(Ct)}. The data shown represent mean ± SD, (n=3).

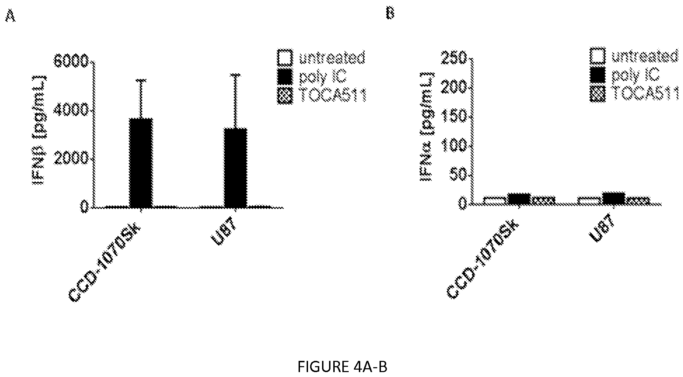

**Figure 4A-B** provides data showing type I IFN production induced by poly (I:C) and Toca 511 in CCD-1070Sk fibroblast and U87-MG glioma cells. Cells were transfected with poly (I:C) at 10 µg/mL or infected with Toca 511 at MOI of 10. Culture supernatant from untreated, poly (I:C) and Toca 511 treated cells were collected 8h post treatment for ELISA to measure the amount of IFNα **(B)** and IPNβ **(A)**, respectively. Data shown represent mean ± SD, (n=3).

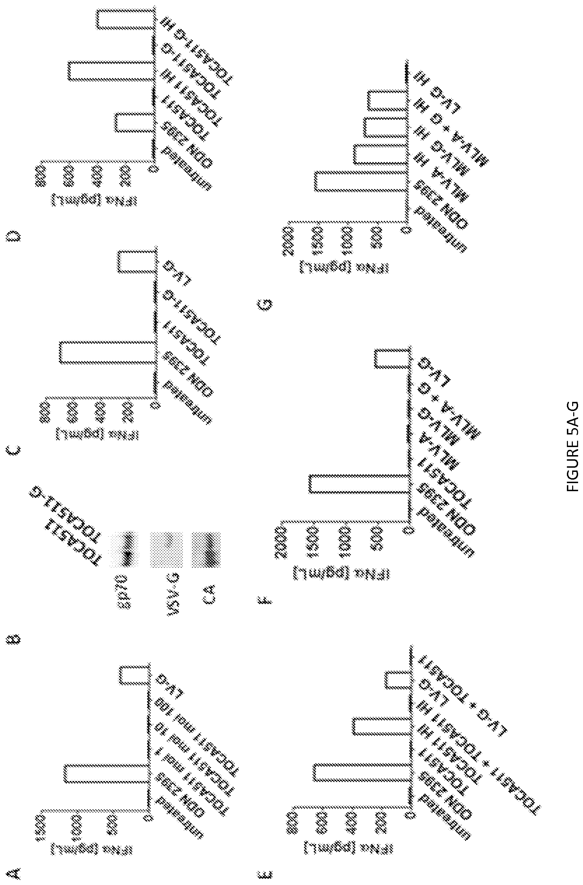

**Figure 5A-I** presents data indicating that IFNα production of pDCs is not induced by MLV-based retroviral vectors unless heat-inactivated, and that MLV-based retroviral vectors can suppress induction of IFNα by either heat-inactivated gamma-retroviral viral vectors such as MLV, or by native lentiviral vectors. **(A)** Toca 511 does not induce IFN production in human pDC. pDCs isolated from healthy individuals were stimulated with ODN2395, Toca 511 at MOI 1, 10 and 100 or lentiviral vector pseudotyped with VSV-G at MOI of 20. Production of IFNα was measured 30 hours after stimulation. **(B)** Western blot of aliquots of Toca 511 and Toca 511 pseudotyped with VSV-G (Toca 511-G) vector preparations developed with antibodies against the amphotropic envelope glycoprotein (gp70), VSV-G protein and the capsid protein (CA). **(C)** Allowing pDC enty of Toca 511 through the VSVg envelope does not lead to IFN production. IFNα production of pDCs stimulated with Toca 511 or Toca 511-G at MOI of 20. **(D)** Heat inactivation of Toca 511 leads to IFN production from human pDCs.IFNα production of pDCs stimulated with Toca 511 or Toca 511-G at MOI of 20 with or without heat inactivation. **(E)** Blockade of IFNα production by pDCs stimulated with co-incubation of Toca 511 and Toca 511 HI (heat inactivated) or Toca 511 and LV-G. **(F)** Non replicative retroviral vectors with amphotropic or VSVg envelopes and no glycogag protein behave like Toca 511. IFNα production of pDCs stimulated with replication defective retroviral vectors pseudotyped with amphotropic envelope protein (MLV-A), VSV-G (MLV-G) and both (MLV-A+G) at MOI of 20. **(G)** Heat inactivation of non replicative retroviral vectors leads to induction of IFN production by pDCs.IFNα production of pDCs stimulated with heat-inactivated MLV-based replication defective retroviral vectors pseudotyped with amphotropic envelope protein (MLV-A), VSV-G (MLV-G) and both (MLV-A+G) at MOI of 20. ODN2395 and lentiviral vector pseudotyped with VSV-G at MOI of 20 were included as positive controls. **(H)** Immunoblots of heat- and non- heat-treated Toca 511 and enzyme digestion with deglycodiases, D; phospholipase C, P; or trypsin, T. **(I)** Blockade by native Toca 511 of induction of IFNα production in pDCs by heat-treated Toca 511,is removed by pre-treatment of native Toca 511 with trypsin (T) but not by pre-treatement of Toca 511 with deglycosylase (D) or phopholipase (PLC) and

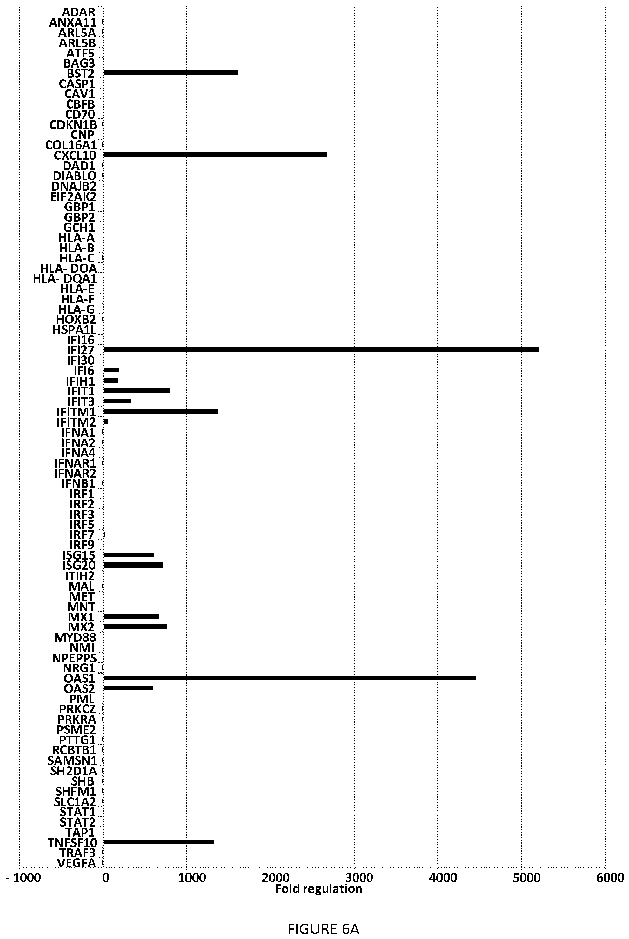

**Figure 6A-B** presents a gene expression profile of the IFNα/β pathway in U87-MG cells. Gene expression profile of U87-MG cells **(A)** treated with IFNβ, or **(B)** infected with Toca 511 at an MOI of 10. Cells were harvested 16 hours post treatment followed by qRT-PCT. Gene expression is presented as fold regulation relative to untreated cells.

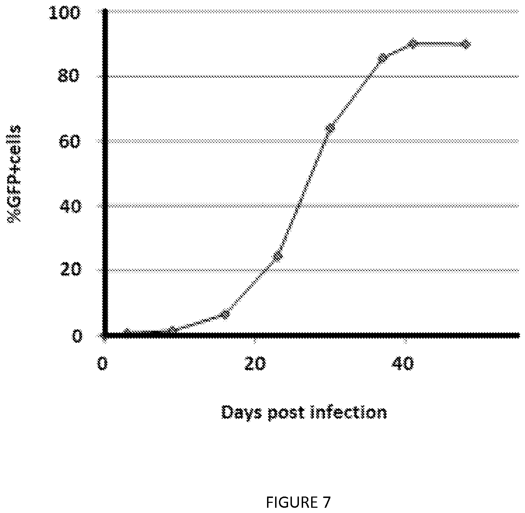

**Figure 7** provides a curve demonstrating the replication kinetics of RRV-GFP in non-tranformed human fibroblast cells. Non-transformed human fibroblast cells (CCD-1070Sk) were infected with RRV-GFP at an MOI of 0.1. Infected cells were passaged ever 3-4 days. A portion of cells at each passage was harvested for measuring percentage of GFP-positive cells.

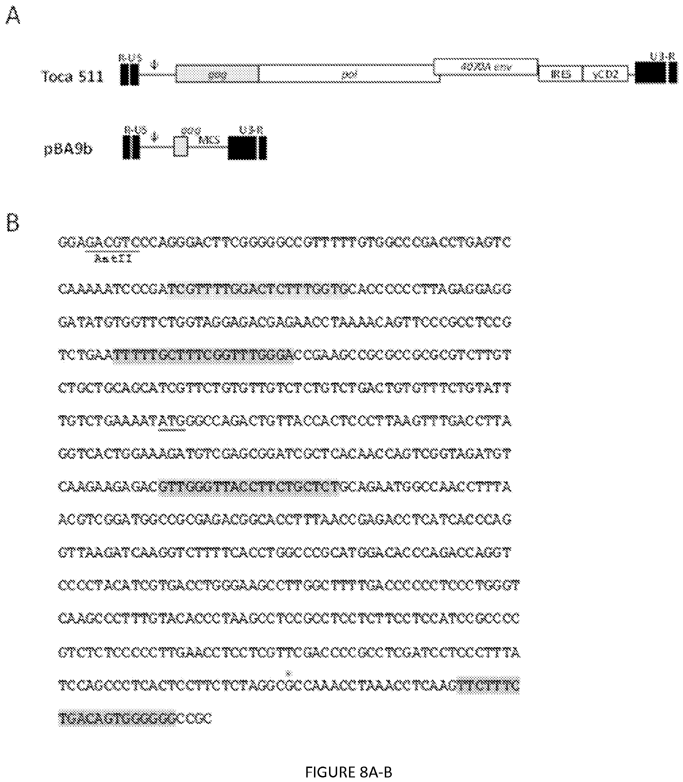

**Figure 8A-B** provides diagrams showing the sequence homology between the Toca 511 viral genome and the pBA9b vector. **(A)** Diagram of sequence overlap between the viral genome of Toca 511 and pBA9b vector. **(B)** Four GU-rich regions are identified in psi and 5' gag in Toca 511 (highlighted), and the first 3 regions are present in the pBA9b vector. Underlined ATG indicates the start codon of gag polypeptide. Asterisk indicates where the gag sequence ends in the pBA9b vector (SEQ ID NO:4).

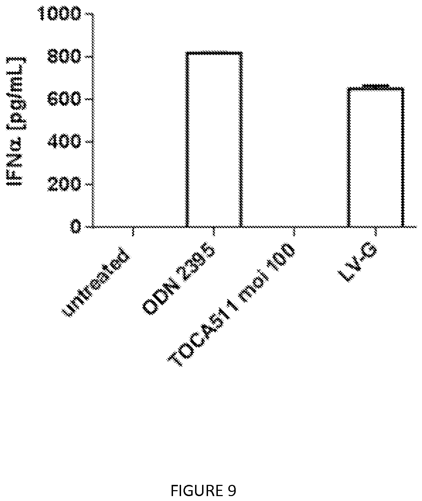

**Figure 9** presents a bar graph demonstrating IFNα production of human pDCs induced by MLV-based retroviral vectors. pDCs isolated from spleens of Balb/c mice were stimulated with ODN2395, Toca 511 at MOI of 100 or lentiviral vector pseudotyped with VSV-G at MOI of 20 (LV-G). Production of IFNα was measured 30 hours after stimulation.

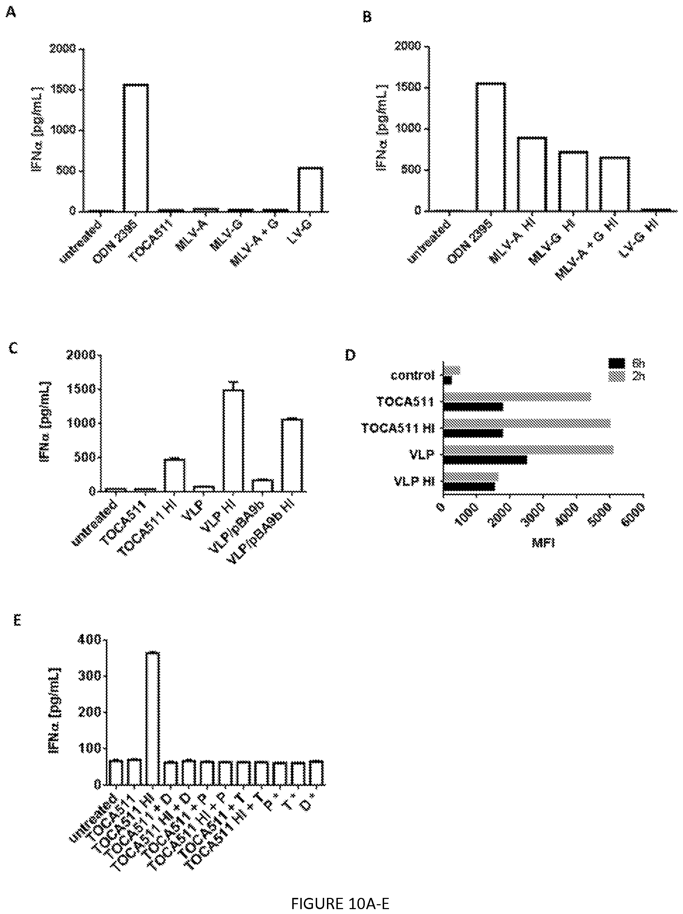

**Figure 10A-E** shows immunosuppressive and immune-stimulating component(s) associated with Toca 511 is independent of the amphotropic envelope glycoprotein and viral nucleic acid. **(A)** No stimulation of IFNα production of pDCs stimulated with replication defective retroviral vectors pseudotyped with amphotropic envelope protein (MLV-A), VSV-G (MLV-G) and both (MLV-A+G) at MOI of 20; ODN2395 and lentiviral vector pseudotyped with VSV-G (LV-G) at moi of 20 are positive controls and do induce IFNα production;Toca 511 is the negative control. **(B)** IFNα production of pDCs stimulated with heat treated retroviral non-replicating vectors pseudotyped with amphotropic envelope protein (MLV-A), VSV-G (MLV-G) and both (MLV-A+G) at MOI of 20. ODN2395 and lentiviral vector pseudotyped with VSV-G at MOI of 20 were included as positive controls. **(C)** Heat treated VLP and VLP/pBA9b induced IFNα production in pDCs, whereas the native versions are not active. **(D)** CFSE-labeled Toca 511 and VLP were added to pDCs at 37 °C to allow for internalization. Cells were harvested at 2 h and 6 h post incubation. Control represents cells incubated with dialyzed dye alone to assess background fluorescence. **(E)** IFNα production of pDCs stimulated with heat- or non-heat-treated Toca 511, with and without enzyme digestion.P=Phopholipase C; D=Deglycosylase; T=Trypsin Asterisk (*) indicates co-incubation of pDCs with enzyme alone.

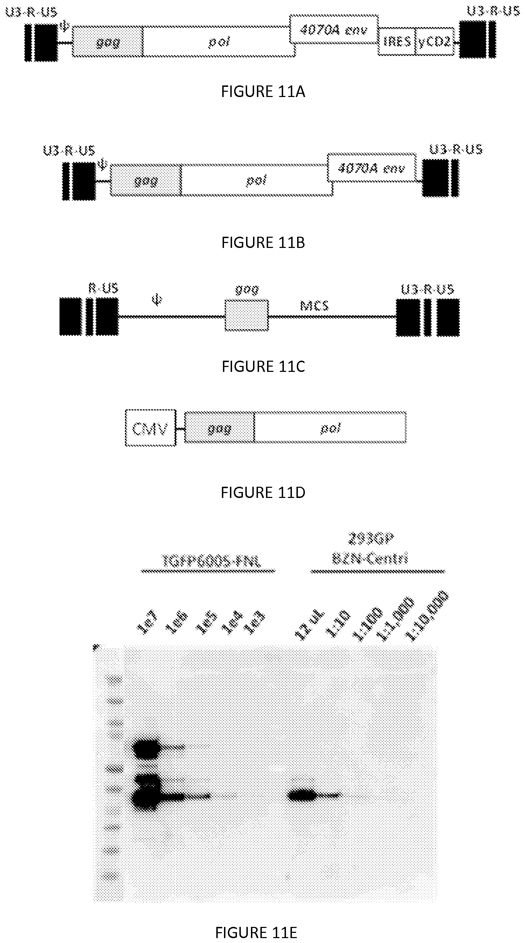

**Figure 11A-E** shows various constructs used in the disclosure. **(A)** A schematic vector map of the MLV-based retroviral replicating vector with IRES-transgene cassette. **(B)** A schematic vector map of MLV-based retroviral replicating vector. **(C)** A schematic vector map of the MLV-based retroviral non-replicating vector. **(D)** A schematic vector map of pCMV-gag-pol for generating MLV-based retroviral virus-like particle (VLPs) in 293GP cells. **(E)** Immunoblot of capsid protein from virus-like particles.

### DETAILED DESCRIPTION

As used herein and in the appended claims, the singular forms "a," "and," and "the" include plural referents unless the context clearly dictates otherwise. Thus, for example, reference to "an immunosuppressive component" includes a plurality of such components and reference to "the retroviral replicating vector" includes reference to one or more retroviral replicating vectors and equivalents thereof known to those skilled in the art, and so forth.

Unless defined otherwise, all technical and scientific terms used herein have the same meaning as commonly understood to one of ordinary skill in the art. Although many methods and reagents similar or equivalent to those described herein, the exemplary methods and materials are presented herein.

All publications mentioned herein are provided for the purpose of describing and disclosing the methodologies, which might be used in connection with the description herein.

Also, the use of "or" means "and/or" unless stated otherwise. Similarly, "comprise," "comprises," "comprising" "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

Retroviruses have been classified in various ways but the nomenclature has been standardized in the last decade (see ICTVdB - The Universal Virus Database, v 4 on the World Wide Web (www) at ncbi.nlm.nih.gov/ICTVdb/ICTVdB/ and the text book "Retroviruses" Eds. Coffin, Hughs and Varmus, Cold Spring Harbor Press 1997). The compositions described herein are obtained or derived from Orthoretroviruses or more typically gammaretroviruses and even more specifically, in certain embodiments, mammalian oncoretroviruses (*e.g*., MLV, GLV, FELV and the like).

As used herein, the term "type I interferon" is defined to include all subtypes of native sequence type I interferons of any mammalian species, including interferon-α, interferon-β, interferon-delta, interferon-Ω and interferon-tau. Similarly, the term "human type I interferon" is defined to include all subtypes of native sequence type I human interferons, including human interferon-α, interferon-β, and interferon-Ω classes and which bind to a common cellular receptor.

Unless otherwise expressly provided, the terms "interferon-α," "IFN-α," and "human interferon-α", "human IFN-α" and "hIFN-α" are used herein to refer to all species of native sequence human alpha interferons, including all subtypes of native sequence human interferon-α. Natural (native sequence) human interferon-α comprises 23 or more closely related proteins encoded by distinct genes with a high degree of structural homology (Weissmann and Weber, Prog. Nucl. Acid. Res. Mol. Biol., 33: 251, 1986; J. Interferon Res., 13: 443-444, 1993; Roberts et al., J. Interferon Cytokine Res. 18: 805-816, 1998). The human IFN-α locus comprises two subfamilies. The first subfamily consists of at least 14 functional, non-allelic genes, including genes encoding IFN-αA (IFN-α2), IFN-αB (IFN-α8), IFN-α (IFN-α10), IFN-αD (IFN-α1), IFN-αE (IFN-α22), IFN-αF (IFN-α21), IFN-αG (IFN-α5), and IFN-αH (IFN-α14), and pseudogenes having at least 80% homology. The second subfamily, α₁₁ or Ω, contains at least 5 pseudogenes and one functional gene (denoted herein as "IFN-α₁₁₁" or "IFN-Ω") which exhibits 70% homology with the IFN-α genes (Weissmann and Weber, 1986. *supra*).

The disclosure provides compositions and methods useful in treating autoimmune disorders by modulating the Type I interferon pathway. The compositions can be used alone or in combination with other interferon modulating compositions.

In general, a potential limitation for replicating viruses as anticancer agents is attaining sufficient viral spread within the tumor mass before immune clearance of the virus. The non-cytolytic nature of retroviral replicating vectors (RRVs) may render them less likely to trigger innate immune responses when compared to oncolytic viruses such as those based on adenovirus or vaccinia virus. RRVs appear to be relatively non-inflammatory, weak immunogens, and, in rodent tumor models, replicate in target tumors without extensive replication elsewhere in healthy tissues. This last property has been demonstrated in brain cancer models in immune-competent rats and mice, and in dog subjects with high grade gliomas. Furthermore, there is rapid elimination of detectable virus upon infusing normal monkeys with amphotropic murine replicating retrovirus preparations. Moreover, these same RRV properties have been observed in humans (*e.g*., clinical trials with Toca 511, Tocagen Inc., San Diego, CA). Toca 511 is an RRV comprising an amphotropic envelope protein and encodes a yeast-derived cytosine deaminase designed to convert 5-fluorocytosine to 5-fluorouracil in infected tumors.

It has been previously speculated that the RRV tumor specificity arises out of a combination of the need to replicate in cell targets for productive infection by gamma-retroviruses and common defects in the cellular innate immune signaling pathways in tumor cells. The innate immune response, besides constituting a direct system of immune defense, is thought to be a necessary precursor to adaptive immunity. Moreover, viral restriction activities, such as APOBEC3G, tetherin and other host restriction factors, are generally downstream effectors induced by the Type I IFNs which are induced by activation of the innate immune signaling pathways through pattern recognition receptors (PRRs).

The disclosure demonstrates that RRVs are less inflammatory and have a relatively attenuated ability to stimulate the innate immune system than was previously known. These RRV properties account, in part, for the lack of viral clearance from tumors by the adaptive immune response and also the permissive virus replication in these tumors.

The innate immune response is predominately mediated by interaction of pathogen-associated molecular patterns (PAMP) with PRRs present on the cell surface or within the intracellular compartments. PRRs which detect viral components include toll-like receptors (TLR2, TLR3, TLR4, TLR7, TLR8 and TLR10), RIG-I-like receptors (RIG-1 and MDA5), PKR, DAI and STING. Upon induction of PRRs, IFNα/β is produced to activate the Type I IFN signaling pathway in an autocrine or paracrine fashion, which subsequently leads to activation of an antiviral state in the cells.

Cells of various types of cancers have defects in the Type I IFN signaling pathway in order to avoid cell death and/or to promote cell proliferation, thereby providing an optimal niche for viral infection and replication within the cells of such cancers. Defects in Type I IFN signaling, including IRF3, and STAT1, as well as deletion in the Type I IFN receptor have been reported in tumor cells, including human glioma cells. The concept that tumor cells develop defects in Type I IFN signaling in order to support tumor growth and that these defects allow replication of viruses which are sensitive to Type I IFN has been demonstrated in oncolytic viruses such as vesicular stomatitis virus (VSV).

This disclosure demonstrates that elements of RRV are useful in modulating Type I IFN-dependent activity. The disclosure provides a coorelation between RRV infection and the Type I IFN-dependent antiviral response in human tumor cells and normal cells. The results presented herein demonstrate that although the replication of RRV is markedly inhibited by exogenous interferon treatment, RRV infection is a less inflammatory event than for other viruses including lentiviral vectors. This data evidences that amphotropic MLV-based RRV do not directly trigger the Type I IFN response in IFN-responsive human glioma tumor-derived cells or cultured non-transformed fibroblast and endothelial cells. The data also provide the first direct evidence that an immunosuppressive component associated with RRV particles actively suppresses the activation of TLRs in plasmacytoid dendritic cells (pDCs). The inhibition of Type I IFN production by RRVs combined with the reduced response to Type I IFN in cancer cells is in agreement with the selective replication of amphotropic MLV-based RRVs in tumors without viral elimination.

The disclosure demonstrates a qualitative difference in interferon responses to RRV compared to other common viral vectors, which is consistent with the observed efficient tumor-specific spread and efficacy in orthotopic glioma mouse models and in glioblastomas in human patients. In the studies described herein, tumor promoting cells (*e.g*., human glioma and other tumor cells, fibroblasts, endothelial cells and pDCs that resemble part of the tumor microenvironment) when infected by viral vectors demonstrated the interplay between MLV-based RRVs and the Type I IFN-dependent antiviral response.

The disclosure futher demonstrates that RRVs are sensitive to exogenous IFNα and β, and that the lower level of responsiveness to gene induction observed in cancer cells (*e.g*., HT1080 and U87-MG) is consistent with the concept that tumor cells have defects in the Type I IFN pathway. However, in studies presented herein, MLV-based RRVs did not elicit a Type I IFN-mediated antiviral response upon viral infection of either tumor cells or non-transformed fibroblasts and primary endothelial cells, and the lack of an IFN-mediated antiviral response is not due to the inability of the cells to produce Type I IFN.

In addition, the disclosure further demonstrates that RRV-GFP did not replicate more efficiently in IFNα/β-resistant cells than the parental cells in the absence of IFNα/β. Thus, more extreme defects in Type I IFN signaling in host cells are not necessary for productive infection with MLV-based RRVs, in these *in vitro* systems, but defects in IFN signaling may play a permissive role in tumors *in vivo.* Also, it is possible that the active suppression of IFN induction observed in human cells may also occurr in fibroblasts and endothelial cells. Of note, recently discovered cytosolic nucleic acid sensors, including HMGBs, TREX1, cGAS and DHX9/36 involved in cellular innate immune response, may also play a role in this blockade.

Although Type I IFNs have been implicated as an important factors for antiviral immunity in the early stages of infection with the Friend strain of MLV (FV). TLR7 and Myd88 may provide a role in mediating the antibody response against retrovirus infection in mice. Activation of the innate response via Type I IFN may be dispensable in resistant mouse strains due to their robust and long-lasting antibody response. The requirement for TLR7 in generating virus-specific anti-MLV antibodies was demonstrated in TLR7-knockout mice. In addition, using IFNaR-knockout model, it was shown that the Type I IFN response was not required to trigger an adaptive immune response in resistant mice. Presumably, in the context of MLV-based retroviral infection, humans may function similarly to the resistant mouse strains, so that the Type I IFNα/β response is dispensable, and that high baseline level of APOBEC3G in resting immune cells control the initial viral replication followed by robust antibody response.

Despite current understanding of the functional role of Myd88 and TLR7 in anti-MLV responses, and the implication that retroviral RNA interacts with TLR7 in an endosomal compartment post-viral entry, it was not found in the studies presented herein that TLR7 in pDCs was activated by replicating or defective retroviral vectors with amphotropic, VSV-G or both envelope glycoproteins unless the viral vectors had been heat-inactivated prior to exposure to pDCs. Intriguingly, the ability of virus-like particle (VLPs) in the absence of the retroviral RNA to induce IFNα production in pDCs suggests that there is another PAMP, in addition to the retroviral RNA, present in Toca 511. Furthermore, the experiments using MLV-based retroviral non-replicating vectors pseudotyped with amphotropic and VSV-G envelope glycoproteins as well as VLPs led to the identification of an additional immunosuppressive component, other than the amphotropic envelope glycoprotein, associated with RRVs. Thus, indicating the presence of an additional PAMP other than viral RNA and an immunosuppressive component associated with MLV-based viral particles. Furthermore, exposure of heat-treated MLV-based viral particles concurrently leads to the exposure of the encrypted PAMP and inactivation of the immunosuppressive component, leading to IFNα production in pDCs. It is also important to note that the MLV-based non-replicating vectors generated in these studies, which are capable of inducing IFNα production in human pDCs under heat treatment, do not express glyco-gag. The N-terminal fragment of glyco-gag expressed from RRV has shown to be incorporated in MLV viral particles and plays a role in inhibiting the function of APOBEC3 in target cells. The data suggests that this APOBEC3 inhibition occurs through a mechanism that is distinct from the Type I IFN-regulated activity. Furthermore, given that both HIV-1 vector and VLPs can induce IFNα production in DCs (presumably mediated via TLR7 and TLR7-independent pathways, respectively), the present data shows that Toca 511 can actively inhibit lentiviral-vector-induced IFNα production in pDCs suggest the inhibition may be through a common downstream component in the TLR7 and TLR7-independent pathways.

As retroviral particles made from different cell types are known to incorporate different cellular proteins, the data suggest that the immunosuppressive and immune-stimulatory components are likely due to viral constituents. This hypothesis is further supported by the results from vectors produced from a canine producer cell line which have similar properties to vectors produced from human cell lines. Further support for viral constituents being the active components for immune suppression as comes from the results showing that co-incubation of amphotropic envelope protein-associated microvesicles produced by transient transfection in 293T and heat-treated VLPs lacking the viral RNA that did not suppress the induction of IFNα production in pDCs. However, it is possible that different host proteins in viral particles produced from different cells can mediate this blockade.

The disclosure also indicates that the replication-defective MLV-based vectors generated in the studies herein, which are capable of inducing IFNα production in human pDCs under heat treatment, do not express glycol-gag (Pr80gag). Accordingly, in another embodiment, Pr80gag plays little to no part in the interferon regulation component(s) associated with RRV viral particles. Furthermore, given that both HIV-1 vector and VLPs can induce IFNα production in DCs (presumably mediated via TLR7 and TLR7-independent pathways, respectively), the data presented herein indicate that Toca 511 can actively inhibit lentiviral-vector-induced IFNα production in pDCs (see **FIG. 5E**) suggesting the inhibition may be through a common downstream component in the TLR7 and TLR7-independent pathways.

The disclosure provides that the Type I IFN response in tumor cells, non-tranformed fibroblasts, primary endothelial cells or pDCs is not induced by amphotropic MLV-based RRV. The findings presented herein, therefore corroborate that cell proliferation is an important determinant in supporting RRV replication, and unlike VSV, other oncolytic viruses and lentiviral vectors, RRVs do not induce cellular innate immune activity in cell cultures. However, the disclosure does not rule out that there may be an *in vivo* mechanism of Type I IFN induction, other than through the cells tested herein, which suppresses viral replication in normal tissue and yet allows for the tumor specific replication observed in some mouse tumor models, and in dogs and humans with gliomas. Moreover, the disclosure indicates that the possible site of the tumor plays an important role in whether or not there is *in vivo* induction of Type I IFN which controls viral replication. Further, many mechanisms have been described in tumors to create immune privilege such as down regulation of adaptive immunity which could contribute to selective replication in tumors.

Based upon the foregoing and the examples below, the disclosure provides for the identification of an immunosuppressive component(s) associated with the RRV particles. The composition comprises a viral like particle (VLP) derived from a gammaretrovirus. The VLP comprises a strutrual and/or functional gag and pol polypeptide. In a particular embodiment, an amphotropic envelope glycoprotein is not the immunosuppressive component associated with the RRV particles. Accordingly, the VLP lacks an envelope. The VLP of the disclosure can be produced by transducing a suitable cell type with a replication defective viral system including a polynucleotide comprising a gag-pol gene operably linked to a heterologous promoter (*e.g*., a CMV promoter; see, *e.g*., **Figure 11D**). The envelope can be removed to give core VLP (K.B. Andersen, Virus Research175:134-142 (2013). Core VLP (Gag self assembled Core VLP cane also be made from bactrial expression and self assembly of gag proteins (Y.Morikawa J. Biol. Chem 279: 31964-31972, 2004)

As used herein, the term "virus-like particle" or "VLP" refers to a nonreplicating, viral shell, derived from any of several viruses discussed further below. VLPs are generally composed of one or more viral proteins, such as, but not limited to those proteins referred to as capsid, coat, shell, surface and/or envelope proteins, or particle-forming polypeptides derived from these proteins. VLPs can form spontaneously upon recombinant expression of the protein in an appropriate expression system. Methods for producing particular VLPs are known in the art and discussed more fully below. The presence of VLPs following recombinant expression of viral proteins can be detected using conventional techniques known in the art, such as by electron microscopy, X-ray crystallography, and the like. See, *e.g*., Baker et al., Biophys. J. (1991) 60:1445-1456; Hagensee et al., J. Virol. (1994) 68:4503-4505. For example, VLPs can be isolated by density gradient centrifugation and/or identified by characteristic density banding. Alternatively, cryoelectron microscopy can be performed on vitrified aqueous samples of the VLP preparation in question, and images recorded under appropriate exposure conditions.

A virus-like particle (VLP) of the disclosure includes a viral particle that includes a gag and pol polypeptide, but is incapable of replication and which, lacks (i) an envelope, and/or (ii) a viral genome (*e.g*., viral nucleic acid). In a specific embodiment, the VLP of the disclosure is a naked viral particle comprising particle-forming polypeptides (*e.g*., gag polypeptides) and can further include pol polypeptides. In another embodiment, the VLP comprises gag and pol polypeptides.

The disclosure provides gag-pol polynucleotide and polypeptide sequences (SEQ ID NO:1 (gag-pol polynucleotide) and SEQ ID NO:2 (gag) and SEQ ID NO:3 (pol), respectively). These gag-pol sequences are exemplary and other gag-pol, gag or pol sequences are readily identifiable by one of skill in the art that are from other gamma retroviruses or which share a high degree of identity and functionality (see, *e.g*., Accession Nos. J02255, M93134, NC001819, NC_001885, and AB67261, each of which provides sequences comprising gag and pol polynucleotides and polypeptide; the sequences associated with each accession number are hereby incorporated herein).

The disclosure provide immuno-supressive compositions comprising (i) a VLP comprising or consisting of SEQ ID NO:2 and/or 3; (ii) a polypeptide comprising or consisting of SEQ ID NO:2 and/or 3; (iii) a VLP comprising or consisting of a polypeptide that has at least 85%, 87%, 90%, 92%, 95%, 98%, 99%, 99.5% identity to SEQ ID NO:2 and/or 3; and (iv) a polypeptide that has at least 85%, 87%, 90%, 92%, 95%, 98%, 99%, 99.5% identity to SEQ ID NO:2 and/or 3 and which immuno-supressive by inhibiting a Type 1 interferon pathway and/or the production of IFNα and/or β.

By "particle-forming polypeptide" derived from a particular viral protein is meant a full-length or near full-length viral protein, as well as a fragment thereof, or a viral protein with internal deletions, which has the ability to form VLPs under conditions that favor VLP formation. Accordingly, the polypeptide may comprise the full-length sequence, fragments, truncated and partial sequences, as well as analogs and precursor forms of the reference molecule. The term therefore intends deletions, additions and substitutions to the sequence, so long as the polypeptide retains the ability to form a VLP and/or suppress the Type I interferon pathway. Thus, the term includes natural variations of the specified polypeptide since variations in coat proteins often occur between viral isolates. The term also includes deletions, additions and substitutions that do not naturally occur in the reference protein, so long as the protein retains the ability to form a VLP and/or suppress the Type I interferon pathway.

The terms "gag polyprotein" or "gag protein", "pro polyprotein" or "pro protein", and "pol polyprotein" or "pol protein" refer to the multiple proteins encoded by retroviral gag, pro, and pol genes which are typically expressed as a single precursor "polyprotein". As used herein, the term "polyprotein" shall include all or any portion of gag, pro, or pol polyproteins. Translation of the viral RNA leads occasionally to synthesis of a fusion protein that is usually called the gag-pol precursor but is now more appropriately called the gag-pro-pol precursor.

A "gag polypeptide" as used herein is the retrovirus-derived structural polypeptide that is responsible for formation of the virus-like particles described herein. In some embodiments, the gag polypeptide may be purposely mutated in order to affect certain characteristics. A typical retroviral genome codes for three major gene products: the gag gene coding for structural proteins, the pol gene coding for reverse transcriptase and associated proteolytic polypeptides, nuclease and integrase associated functions, and env whose encoded glycoprotein membrane proteins are detected on the surface of infected cells and also on the surface of mature released virus particles. The gag genes of all retroviruses have an overall structural similarity and within each group of retroviruses are conserved at the amino acid level. The gag gene gives rise to the core proteins excluding the reverse transcriptase.

For MLV the gag precursor polyprotein is Pr65^{Gag} and is cleaved into four proteins whose order on the precursor is NH₂-p15-pp12-p30-p10-COOH. These cleavages are mediated by a viral protease and may occur before or after viral release depending upon the virus. The MLV gag protein exists in a glycosylated and a non-glycosylated form. The glycosylated forms are cleaved from gPr80^{Gag} which is synthesized from a different inframe initiation codon located upstream from the AUG codon for the non-glycosylated Pr65^{Gag}. Deletion mutants of MLV that do not synthesize the glycosylated Gag are still infectious and the non-glycosylated Gag can still form virus-like particles.

Structurally, the prototypical gag polyprotein is divided into three main proteins that generally occur in the same order in retroviral *gag* genes: the matrix protein (MA), the capsid protein (CA), and the nucleocapsid protein (NC). Processing of the gag polyprotein into the mature proteins is catalyzed by the retroviral encoded protease and occurs as the newly budded viral particles mature. Functionally, the gag polyprotein is divided into three domains: the membrane binding domain, which targets the gag polyprotein to the cellular membrane; the interaction domain which promotes gag polymerization; and the late domain which facilitates release of nascent virions from the host cell. The form of the gag protein that mediates assembly is the polyprotein. The gag polypeptide as included herein therefore includes the functional elements for formation and release of the VLPs. Those of skill in the art will readily undertand the purposes, the cloning, expression and sequences associated with gag polypeptides and polynucleotides (see, *e.g*., Hansen et al., J. Virol., 64:5306-5316, 1990; Will et al., AIDS, 5:639-654, 1991; Wang et al., J. Virol., 72:7950-7959, 1998; McDonnell et al., J. Mol. Biol., 279:921-928, 1998; Schultz and Rein, J. Virol., 63:2370-2372, 1989; Accola et al., J. Virol., 72:2072-2078, 1998; Borsetti et al., J. Virol., 72:9313-9317, 1998; Bowzard et al., J. Virol., 72:9034-9044, 1998; Krishna et al., J. Virol., 72:564-577, 1998; Wills et al., J. Virol., 68:6605-6618, 1994; Xiang et al., J. Virol., 70:5695-5700, 1996; and Garnier et al., J. Virol., 73:2309-2320, 1999).

As used in the VLPs of the disclosure, the gag polypeptide can include the functional elements for formation of the VLP. The gag polypeptide may optionally include one or more additional polypeptides that may be generated by splicing the coding sequence for the one or more additional polypeptides into the gag polypeptide coding sequence. A useful site for insertion of additional polypeptides into the gag polypeptide is the C-terminus.

The pol polypeptide of the disclosure are known in the art. The pol gene encodes the RNA-directed DNA polymerase (reverse transcriptase), protease and integrase proteins. It has been demonstrated the the pol protein can be incorporated in to the viral capsid alone although at a lower rate (*e.g*., not as a gag-pol polyprotein but as a pol protein).

Methods of determine the percent identity, relatedness, homology and the like are known in the art. The following terms are used to describe the sequence relationships between two or more nucleic acids or polynucleotides: (a) "reference sequence," (b) "comparison window," (c) "sequence identity," (d) "percentage of sequence identity," and (e) "substantial identity."

As used herein, "reference sequence" is a defined sequence used as a basis for sequence comparison. A reference sequence may be a subset or the entirety of a specified sequence; for example, as a segment of a full-length cDNA or gene sequence, or the complete cDNA or gene sequence.

As used herein, "comparison window" makes reference to a contiguous and specified segment of a polynucleotide sequence, wherein the polynucleotide sequence in the comparison window may comprise additions or deletions (*i.e*., gaps) compared to the reference sequence (which does not comprise additions or deletions) for optimal alignment of the two sequences. Generally, the comparison window is at least 20 contiguous nucleotides in length, and optionally can be 30, 40, 50, 100, or longer. Those of skill in the art understand that to avoid a high similarity to a reference sequence due to inclusion of gaps in the polynucleotide sequence a gap penalty is typically introduced and is subtracted from the number of matches.

Methods of alignment of sequences for comparison are well-known in the art. Thus, the determination of percent identity between any two sequences can be accomplished using a mathematical algorithm. Non-limiting examples of such mathematical algorithms are the algorithm of Myers and Miller (Myers and Miller, CABIOS, 4, 11 (1988)); the local homology algorithm of Smith *et al.* (Smith et al., Adv. Appl. Math., 2, 482 (1981)); the homology alignment algorithm of Needleman and Wunsch (Needleman and Wunsch, JMB, 48, 443 (1970)); the search-for-similarity-method of Pearson and Lipman (Pearson and Lipman, Proc. Natl. Acad. Sci. USA, 85, 2444 (1988)); the algorithm of Karlin and Altschul (Karlin and Altschul, Proc. Natl. Acad. Sci. USA, 87, 2264 (1990)), modified as in Karlin and Altschul (Karlin and Altschul, Proc. Natl. Acad. Sci. USA 90, 5873 (1993)).

Computer implementations of these mathematical algorithms can be utilized for comparison of sequences to determine sequence identity. Such implementations include, but are not limited to: CLUSTAL in the PC/Gene program (available from Intelligenetics, Mountain View, Calif.); the ALIGN program (Version 2.0) and GAP, BESTFIT, BLAST, FASTA, and TFASTA in the Wisconsin Genetics Software Package, Version 8 (available from Genetics Computer Group (GCG), 575 Science Drive, Madison, Wis., USA). Alignments using these programs can be performed using the default parameters. The CLUSTAL program is well described by Higgins *et al.* (Higgins et al., CABIOS, 5, 151 (1989)); Corpet *et al.* (Corpet et al., Nucl. Acids Res., 16, 10881 (1988)); Huang *et al.* (Huang et al., CABIOS, 8, 155 (1992)); and Pearson *et al.* (Pearson et al., Meth. Mol. Biol., 24, 307 (1994)). The ALIGN program is based on the algorithm of Myers and Miller, supra. The BLAST programs of Altschul *et al.* (Altschul et al., JMB, 215, 403 (1990)) are based on the algorithm of Karlin and Altschul supra.

Software for performing BLAST analyses is publicly available through the National Center for Biotechnology Information. This algorithm involves first identifying high scoring sequence pairs (HSPs) by identifying short words of length "W" in the query sequence, which either match or satisfy some positive-valued threshold score T when aligned with a word of the same length in a database sequence. "T" is referred to as the neighborhood word score threshold. These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters "M" (reward score for a pair of matching residues; always >0) and "N" (penalty score for mismatching residues; always <0). For amino acid sequences, a scoring matrix is used to calculate the cumulative score. Extension of the word hits in each direction are halted when the cumulative alignment score falls off by the quantity "X" from its maximum achieved value, the cumulative score goes to zero or below due to the accumulation of one or more negative-scoring residue alignments, or the end of either sequence is reached.

In addition to calculating percent sequence identity, the BLAST algorithm also performs a statistical analysis of the similarity between two sequences. One measure of similarity provided by the BLAST algorithm is the smallest sum probability (P(N)), which provides an indication of the probability by which a match between two nucleotide or amino acid sequences would occur by chance. For example, a test nucleic acid sequence is considered similar to a reference sequence if the smallest sum probability in a comparison of the test nucleic acid sequence to the reference nucleic acid sequence is less than about 0.1, less than about 0.01, or even less than about 0.001.

To obtain gapped alignments for comparison purposes, Gapped BLAST (in BLAST 2.0) can be utilized. Alternatively, PSI-BLAST (in BLAST 2.0) can be used to perform an iterated search that detects distant relationships between molecules. When utilizing BLAST, Gapped BLAST, PSI-BLAST, the default parameters of the respective programs (*e.g*., BLASTN for nucleotide sequences, BLASTX for proteins) can be used. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix. Alignment may also be performed manually by inspection.

For purposes of the disclosure, comparison of nucleotide sequences for determination of percent sequence identity to the promoter sequences disclosed herein may be made using the BlastN program (version 1.4.7 or later) with its default parameters or any equivalent program. By "equivalent program" is intended any sequence comparison program that, for any two sequences in question, generates an alignment having identical nucleotide or amino acid residue matches and an identical percent sequence identity when compared to the corresponding alignment generated by the program.

As used herein, "sequence identity" or "identity" in the context of two nucleic acid or polypeptide sequences makes reference to a specified percentage of residues in the two sequences that are the same when aligned for maximum correspondence over a specified comparison window, as measured by sequence comparison algorithms or by visual inspection. When percentage of sequence identity is used in reference to proteins it is recognized that residue positions which are not identical often differ by conservative amino acid substitutions, where amino acid residues are substituted for other amino acid residues with similar chemical properties (*e.g*., charge or hydrophobicity) and therefore do not change the functional properties of the molecule. When sequences differ in conservative substitutions, the percent sequence identity may be adjusted upwards to correct for the conservative nature of the substitution. Sequences that differ by such conservative substitutions are said to have "sequence similarity" or "similarity." Means for making this adjustment are well known to those of skill in the art. Typically this involves scoring a conservative substitution as a partial rather than a full mismatch, thereby increasing the percentage sequence identity. Thus, for example, where an identical amino acid is given a score of 1 and a non-conservative substitution is given a score of zero, a conservative substitution is given a score between zero and 1. The scoring of conservative substitutions is calculated, *e.g*., as implemented in the program PC/GENE (Intelligenetics, Mountain View, Calif.).

As used herein, "percentage of sequence identity" means the value determined by comparing two optimally aligned sequences over a comparison window, wherein the portion of the polynucleotide sequence in the comparison window may comprise additions or deletions (*i.e*., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison, and multiplying the result by 100 to yield the percentage of sequence identity.

The term "substantial identity" of polynucleotide sequences means that a polynucleotide comprises a sequence that has at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, or 94%, or even at least 95%, 96%, 97%, 98%, or 99% sequence identity, compared to a reference sequence (*e.g*., SEQ ID NO:1) using one of the alignment programs described using standard parameters. One of skill in the art will recognize that these values can be appropriately adjusted to determine corresponding identity of proteins encoded by two nucleotide sequences by taking into account codon degeneracy, amino acid similarity, reading frame positioning, and the like. Substantial identity of amino acid sequences for these purposes normally means sequence identity of at least 70%, 80%, 90%, or even at least 95%.

Another indication that nucleotide sequences are substantially identical is if two molecules hybridize to each other under stringent conditions. Generally, stringent conditions are selected to be about 5 °C lower than the thermal melting point (Tm) for the specific sequence at a defined ionic strength and pH. However, stringent conditions encompass temperatures in the range of about 1°C to about 20°C, depending upon the desired degree of stringency as otherwise qualified herein. Nucleic acids that do not hybridize to each other under stringent conditions are still substantially identical if the polypeptides they encode are substantially identical. This may occur, *e.g*., when a copy of a nucleic acid is created using the maximum codon degeneracy permitted by the genetic code. One indication that two nucleic acid sequences are substantially identical is when the polypeptide encoded by the first nucleic acid is immunologically cross reactive with the polypeptide encoded by the second nucleic acid.

The term "substantial identity" in the context of a peptide indicates that a peptide comprises a sequence with at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, or 94%, or even 95%, 96%, 97%, 98% or 99%, sequence identity to the reference sequence over a specified comparison window. In certain embodiments, optimal alignment is conducted using the homology alignment algorithm of Needleman and Wunsch (Needleman and Wunsch, JMB, 48, 443 (1970)). An indication that two peptide sequences are substantially identical is that one peptide is immunologically reactive with antibodies raised against the second peptide. Thus, a peptide is substantially identical to a second peptide, for example, where the two peptides differ only by a conservative substitution. Thus, the invention also provides nucleic acid molecules and peptides that are substantially identical to the nucleic acid molecules and peptides presented herein.

For sequence comparison, typically one sequence acts as a reference sequence to which test sequences are compared. When using a sequence comparison algorithm, test and reference sequences are input into a computer, subsequence coordinates are designated if necessary, and sequence algorithm program parameters are designated. The sequence comparison algorithm then calculates the percent sequence identity for the test sequence(s) relative to the reference sequence, based on the designated program parameters.

As noted above, another indication that two nucleic acid sequences are substantially identical is that the two molecules hybridize to each other under stringent conditions. The phrase "hybridizing specifically to" refers to the binding, duplexing, or hybridizing of a molecule only to a particular nucleotide sequence under stringent conditions when that sequence is present in a complex mixture (*e.g*., total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target nucleic acid sequence.

"Stringent hybridization conditions" and "stringent hybridization wash conditions" in the context of nucleic acid hybridization experiments such as Southern and Northern hybridizations are sequence dependent, and are different under different environmental parameters. Longer sequences hybridize specifically at higher temperatures. The thermal melting point (Tm) is the temperature (under defined ionic strength and pH) at which 50% of the target sequence hybridizes to a perfectly matched probe. Specificity is typically the function of post-hybridization washes, the critical factors being the ionic strength and temperature of the final wash solution. For DNA-DNA hybrids, the Tm can be approximated from the equation of Meinkoth and Wahl (1984); Tm 81.5°C +16.6 (log M)+0.41 (% GC)-0.61 (% form)-500/L; where M is the molarity of monovalent cations, % GC is the percentage of guanosine and cytosine nucleotides in the DNA, % form is the percentage of formamide in the hybridization solution, and L is the length of the hybrid in base pairs. Tm is reduced by about 1°C for each 1% of mismatching; thus, Tm, hybridization, and/or wash conditions can be adjusted to hybridize to sequences of the desired identity. For example, if sequences with >90% identity are sought, the Tm can be decreased 10°C. Generally, stringent conditions are selected to be about 5°C lower than the Tm for the specific sequence and its complement at a defined ionic strength and pH. However, severely stringent conditions can utilize a hybridization and/or wash at 1, 2, 3, or 4°C lower than the Tm; moderately stringent conditions can utilize a hybridization and/or wash at 6, 7, 8, 9, or 10°C lower than the Tm; low stringency conditions can utilize a hybridization and/or wash at 11, 12, 13, 14, 15, or 20°C lower than the T.sub.m. Using the equation, hybridization and wash compositions, and desired temperature, those of ordinary skill will understand that variations in the stringency of hybridization and/or wash solutions are inherently described. If the desired degree of mismatching results in a temperature of less than 45°C (aqueous solution) or 32°C (formamide solution), the SSC concentration is increased so that a higher temperature can be used. Generally, highly stringent hybridization and wash conditions are selected to be about 5°C lower than the Tm for the specific sequence at a defined ionic strength and pH.

An example of highly stringent wash conditions is 0.15 M NaCl at 72°C for about 15 minutes. An example of stringent wash conditions is a 0.2xSSC wash at 65°C for 15 minutes. Often, a high stringency wash is preceded by a low stringency wash to remove background probe signal. An example medium stringency wash for a duplex of, *e.g*., more than 100 nucleotides, is 1xSSC at 45°C for 15 minutes. For short nucleotide sequences (*e.g*., about 10 to 50 nucleotides), stringent conditions typically involve salt concentrations of less than about 1.5 M, less than about 0.01 to 1.0 M, Na ion concentration (or other salts) at pH 7.0 to 8.3, and the temperature is typically at least about 30°C and at least about 60°C for long probes (*e.g*., >50 nucleotides). Stringent conditions may also be achieved with the addition of destabilizing agents such as formamide. In general, a signal to noise ratio of 2x (or higher) than that observed for an unrelated probe in the particular hybridization assay indicates detection of a specific hybridization. Nucleic acids that do not hybridize to each other under stringent conditions are still substantially identical if the proteins that they encode are substantially identical. This occurs, *e.g*., when a copy of a nucleic acid is created using the maximum codon degeneracy permitted by the genetic code.

Very stringent conditions are selected to be equal to the Tm for a particular probe. An example of stringent conditions for hybridization of complementary nucleic acids that have more than 100 complementary residues on a filter in a Southern or Northern blot is 50% formamide, *e.g*., hybridization in 50% formamide, 1 M NaCl, 1% SDS at 37°C, and a wash in 0.1xSSC at 60 to 65°C. Exemplary low stringency conditions include hybridization with a buffer solution of 30 to 35% formamide, 1 M NaCl, 1% SDS (sodium dodecyl sulphate) at 37°C, and a wash in 1x to 2xSSC (20xSSC=3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5x to 1xSSC at 55 to 60°C.

The disclosure provides compositions comprising a gamma-retroviral derived composition that is not capable of replication and that suppresses IFN production and/or suppresses a Type I IFN pathway. Disclosed herein, the composition comprises a viral like particle comprising or consisting of a gag and pol polypeptide from a gamma retrovirus such as MLV. Also disclosed herein, the VLP consists of a viral particle and a gag and pol polypeptide. In another embodiment, the composition comprises gag and pol polypeptide from a gamma retrovirus.

The compositions of the disclosure are for use in the treatment of immune disorders. As used herein, the term "immune disorder" and like terms means a disease, disorder or condition caused by the immune system of an animal, including autoimmune disorders. Immune disorders include those diseases, disorders or conditions that have an immune component and those that are substantially or entirely immune system-mediated. In one embodiment, the immune disease or disorder is a Type I interferon mediated immune disease or disorder. In another embodiment, the immune disease or disorder is associated with IFNα. Autoimmune disorders are those wherein the individual's own immune system mistakenly attacks self, thereby targeting the cells, tissues, and/or organs of the individual's own body. For example, the autoimmune reaction is directed against the nervous system in multiple sclerosis and the gut in Crohn's disease. In other autoimmune disorders such as systemic lupus erythematosus (lupus), affected tissues and organs may vary among individuals with the same disease. One person with lupus may have affected skin and joints whereas another may have affected skin, kidney, and lungs. Ultimately, damage to certain tissues by the immune system may be permanent, as with destruction of insulin-producing cells of the pancreas in Type 1 diabetes mellitus. Specific autoimmune disorders that may be ameliorated using the compounds and methods of this invention include without limitation, autoimmune disorders of the nervous system (*e.g*., multiple sclerosis, myasthenia gravis, autoimmune neuropathies such as Guillain-Barre, and autoimmune uveitis), autoimmune disorders of the blood (*e.g*., autoimmune hemolytic anemia, pernicious anemia, and autoimmune thrombocytopenia), autoimmune disorders of the blood vessels (*e.g*., temporal arteritis, anti-phospholipid syndrome, vasculitides such as Wegener's granulomatosis, and Behcet's disease), autoimmune disorders of the skin (*e.g*., psoriasis, dermatitis herpetiformis, pemphigus vulgaris, and vitiligo), autoimmune disorders of the gastrointestinal system (*e.g*., Crohn's disease, ulcerative colitis, primary biliary cirrhosis, and autoimmune hepatitis), autoimmune disorders of the endocrine glands (*e.g*., Type 1 or immune-mediated diabetes mellitus, Grave's disease. Hashimoto's thyroiditis, autoimmune oophoritis and orchitis, and autoimmune disorder of the adrenal gland); and autoimmune disorders of multiple organs (including connective tissue and musculoskeletal system diseases) (*e.g*., rheumatoid arthritis, systemic lupus erythematosus, scleroderma, polymyositis, dermatomyositis, spondyloarthropathies such as ankylosing spondylitis, and Sjogren's syndrome). In addition, other immune system mediated diseases, such as graft-versus-host disease and allergic disorders, are also included in the definition of immune disorders herein. Because a number of immune disorders are caused by inflammation, there is some overlap between disorders that are considered immune disorders and inflammatory disorders. For the purpose of this disclosure, in the case of such an overlapping disorder, it may be considered either an immune disorder or an inflammatory disorder. "Treatment of an immune disorder" herein refers to administering a compound or a composition of the disclosure to a subject, who has an immune disorder, a symptom of such a disease or a predisposition towards such a disease, with the purpose to cure, relieve, alter, affect, or prevent the autoimmune disorder, the symptom of it, or the predisposition towards it.

Disclosed herein the compounds of this disclosure are for use in the treatment or prevention of inflammatory disorders. As used herein, an "inflammatory disorder" means a disease, disorder or condition characterized by inflammation of body tissue or having an inflammatory component. These include local inflammatory responses and systemic inflammation. Examples of such inflammatory disorders include: transplant rejection, including skin graft rejection; chronic inflammatory disorders of the joints, including arthritis, rheumatoid arthritis, osteoarthritis and bone diseases associated with increased bone resorption; inflammatory bowel diseases such as ileitis, ulcerative colitis, Barrett's syndrome, and Crohn's disease; inflammatory lung disorders such as asthma, adult respiratory distress syndrome, and chronic obstructive airway disease; inflammatory disorders of the eye including corneal dystrophy, trachoma, onchocerciasis, uveitis, sympathetic ophthalmitis and endophthalmitis; chronic inflammatory disorders of the gums, including gingivitis and periodontitis; tuberculosis; leprosy; inflammatory diseases of the kidney including uremic complications, glomerulonephritis and nephrosis; inflammatory disorders of the skin including sclerodermatitis, psoriasis and eczema; inflammatory diseases of the central nervous system, including chronic demyelinating diseases of the nervous system, multiple sclerosis, AIDS-related neurodegeneration and Alzheimer's disease, infectious meningitis, encephalomyelitis, Parkinson's disease, Huntington's disease, amyotrophic lateral sclerosis and viral or autoimmune encephalitis; autoimmune disorders, immune-complex vasculitis, systemic lupus and erythematodes; systemic lupus erythematosus (SLE); and inflammatory diseases of the heart such as cardiomyopathy, ischemic heart disease hypercholesterolemia, atherosclerosis); as well as various other diseases with significant inflammatory components, including preeclampsia; chronic liver failure, brain and spinal cord trauma, cancer). There may also be a systemic inflammation of the body, exemplified by gram-positive or gram negative shock, hemorrhagic or anaphylactic shock, or shock induced by cancer chemotherapy in response to pro-inflammatory cytokines, *e.g*., shock associated with pro-inflammatory cytokines. Such shock can be induced, *e.g*., by a chemotherapeutic agent used in cancer chemotherapy.

"Treatment of an inflammatory disorder" herein refers to administering a compound or a composition of the invention to a subject, who has an inflammatory disorder, a symptom of such a disorder or a predisposition towards such a disorder, with the purpose to cure, relieve, alter, affect, or prevent the inflammatory disorder, the symptom of it, or the predisposition towards it.

The methods for immunosuppression or for treating or preventing inflammatory conditions and immune disorders in a patient in need thereof can further comprise administering to the patient being administered a composition of this disclosure (*e.g*., a composition comprising a VLPs, viral particle containing gag-pol polypeptides, and the like, at does ranging from lOng of p30 protein if a administered locally, to 1g if administered by the intravenous route; intermediate doses may also be used depending on the indication being treated, the purpose of the treatment and/or the route of administration, see below), and can futher include an effective amount of one or more other active agents. Such active agents may include those used conventionally for immunosuppression or for inflammatory conditions or immune disorders. These other active agents may also be those that provide other benefits when administered in combination with the compositions of the disclosure. For example, other therapeutic agents may include, without limitation, steroids, non-steroidal anti-inflammatory agents, antihistamines, analgesics, immunosuppressive agents and suitable mixtures thereof. In such combination therapy treatment, both the composition of this disclosure and the other drug agent(s) are administered to a subject (*e.g*., a mammal, human etc.; male or female) by conventional methods. The agents may be administered in a single dosage form or in separate dosage forms. Effective amounts of the other therapeutic agents and dosage forms are well known to those skilled in the art. It is well within the skilled artisan's purview to determine the other therapeutic agent's optimal effective-amount range.

Where another therapeutic agent is administered to a subject, the effective amount of the composition of the disclosure is less than its effective amount when the other therapeutic agent is not administered. Disclosed herein the effective amount of the conventional agent is less than its effective amount when the composition of the disclosure is not administered. In this way, undesired side effects associated with high doses of either agent may be minimized. Other potential advantages (including without limitation improved dosing regimens and/or reduced drug cost) will be apparent to those of skill in the art.

Disclosed herein, relating to autoimmune and inflammatory conditions, the other therapeutic agent may be a steroid or a non-steroidal anti-inflammatory agent. Particularly useful non-steroidal anti-inflammatory agents, include, but are not limited to, aspirin, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam; salicylic acid derivatives, including aspirin, sodium salicylate, choline magnesium trisalicylate, salsalate, diflunisal, salicylsalicylic acid, sulfasalazine, and olsalazin; para-aminophennol derivatives including acetaminophen and phenacetin; indole and indene acetic acids, including indomethacin, sulindac, and etodolac; heteroaryl acetic acids, including tolmetin, diclofenac, and ketorolac; anthranilic acids (fenamates), including mefenamic acid, and meclofenamic acid; enolic acids, including oxicams (piroxicam, tenoxicam), and pyrazolidinediones (phenylbutazone, oxyphenthartazone); and alkanones, including nabumetone and pharmaceutically acceptable salts thereof and mixtures thereof. For a more detailed description of the NSAIDs, see Paul A. Insel, Analgesic-Antipyretic and Antiinflammatory Agents and Drugs Employed in the Treatment of Gout, in Goodman & Gilman's The Pharmacological Basis of Therapeutics 617-57 (Perry B. Molinhoff and Raymond W. Ruddon eds., 9.sup.th ed 1996) and Glen R. Hanson, Analgesic, Antipyretic and Anti-Inflammatory Drugs in Remington: The Science and Practice of Pharmacy Vol II 1196-1221 (A. R. Gennaro ed. 19th ed. 1995).

Immunosuppressive agents include glucocorticoids, corticosteroids (such as Prednisone or Solumedrol), T cell blockers (such as cyclosporin A and FK506), purine analogs (such as azathioprine (Imuran)), pyrimidine analogs (such as cytosine arabinoside), alkylating agents (such as nitrogen mustard, phenylalanine mustard, buslfan, and cyclophosphamide), folic acid antagonsists (such as aminopterin and methotrexate), antibiotics (such as rapamycin, actinomycin D, mitomycin C, puramycin, and chloramphenicol), human IgG, antilymphocyte globulin (ALG), and antibodies (such as anti-CD3 (OKT3), anti-CD4 (OKT4), anti-CD5, anti-CD7, anti-IL-2 receptor, anti-alpha/beta TCR, anti-ICAM-1, anti-CD20 (Rituxan), anti-IL-12 and antibodies to immunotoxins).

The foregoing and other useful combination therapies will be understood and appreciated by those of skill in the art. Potential advantages of such combination therapies include a different efficacy profile, the ability to use less of each of the individual active ingredients to minimize toxic side effects, synergistic improvements in efficacy, improved ease of administration or use and/or reduced overall expense of compound preparation or formulation.

In one embodiment, a VLP of the disclosure can be administered in combination with a steroidal composition. Disclosed herein, the VLP alone or in combination with a steroid may be administered to a subject prior to administration of a viral vector of gene therapy or gene delivery. Disclosed herein, the VLP alone or in combination with a steroid or other immunosuppressive composition is administered prior to delivery of Toca 511 (see, U.S. Pat. No. 8,722,867, In this way the immune response to Toca 511 is suppressed.

The term "effective amount" refers to an amount of an active ingredient of a composition of the disclosure (*e.g*., a VLP) effective to treat (including prevention) of a disease, disorder or unwanted physiological conditions in a mammal (*e.g*., Type I interferon pathway activation, IFNα production etc.). In the disclosure, an "effective amount" of a composition fo the dislcosure may reduce, slow down or delay an autoimmune disorder such as IDDM or SLE; reduce, prevent or inhibit (*i.e*., slow to some extent and preferably stop) the development of an autoimmune disorder such as IDDM or SLE; and/or relieve to some extent one or more of the symptoms associated with autoimmune disorders such as IDDM or SLE.

In the methods of the disclosure, the term "control" and grammatical variants thereof, are used to refer to the prevention, partial or complete inhibition, reduction, delay or slowing down of an unwanted event, *e.g*. physiological condition, such as the generation of autoreactive T cells and development of autoimmunity.

"Treatment" refers to both therapeutic treatment and prophylactic or preventative measures. Those in need of treatment include those already with the disorder as well as those prone to have the disorder or those in which the disorder is to be prevented. For purposes of this disclosure, beneficial or desired clinical results include, but are not limited to, alleviation of symptoms, diminishment of extent of disease, stabilized (*i.e*., not worsening) state of disease, delay or slowing of disease progression, amelioration or palliation of the disease state, and remission (whether partial or total), whether detectable or undetectable.

"Treatment" can also mean prolonging survival as compared to expected survival if not receiving treatment. Those in need of treatment include those already with the condition or disorder as well as those prone to have the condition or disorder or those in which the condition or disorder is to be prevented.

"Pharmaceutically acceptable" carriers, excipients, or stabilizers are ones which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN, polyethylene glycol (PEG), and PLURONICS.

"Mammal" for purposes of treatment refers to any animal classified as a mammal, including humans, domestic and farm animals, and zoo, sports, or pet animals, such as dogs, horses, cats, cows, etc. Typically, the mammal is human.

The disclosure provides a method for the treatment of a disease or condition associated with the expression of IFN-α in a patient, comprising administering to the patient an effective amount of a VLP or gammaretrovirus-derived composition that is immunosuppressive. The administration can include additional immunosuppressive agents such as, e.g., an anti-IFN-a antibody. The patient is a mammalian patient, typically a human patient. The disease is an autoimmune disease, such as insulin-dependent diabetes mellitus (IDDM); systemic lupus erythematosus (SLE); or autoimmune thyroiditis.

The following examples are intended to illustrate but not limit the disclosure. While they are typical of those that might be used, other procedures known to those skilled in the art may alternatively be used.

### EXAMPLES

**Materials.** The following cell lines were obtained from American Type Culture Collection (ATCC): human fibrosarcoma cells (HT1080); human astrocytoma cells U87-MG (HTB-14); human prostate adenocarcinoma cells PC3 (CRL-1435); human prostate carcinoma cells, LN-CaP (CRL-1740); human T-lymphocytes Sup-T1 (CRL-1942); human fibroblast cell lines WI-38 (CCL-75) and CCD-1070Sk (CRL-2091) and canine cells derived from normal fetal thymus Cf2Th (CRL-1430). Primary HUVEC were obtained from Vec Technologies. 293T cells were obtained through noncommercial sources. 293GP cells, which express high levels of Moloney MLV gag and pol as described in Friedmann et al. (Proc Natl Acad Sci USA 90:8033-8037, 1993). 293GP/pBA9b cells, which contain an integrated pBA9b provirus, are derived from 239GP cells which were transduced with VSV-G pseudotyped MLV vector.

**Cell culture.** 293T, 293GP, 293GP/pBA9b, HT1080, U87-MG, PC3 and Cf2Th cells were cultured in complete DMEM medium containing 10% FBS (Hyclone), sodium pyruvate, glutamax, and penicillin/streptomycin and antibiotics (penicillin 100 IU/mL, streptomycin 50 µg/mL). LN-CaP and Sup-T1 cells were cultured in complete RPMI medium containing 10% FBS, Glutamax and penicillin/streptomycin. WI-38 cells were cultured in MEM containing 10% FBS, Glutamax and penicillin/streptomycin. Primary HUVEC were cultured in MCDB-131C medium (VecTechnologies).

Experiments in which U78-MG cells were treated with exogenous Type I IFN, recombinant IFNα2a or recombinant IPNβ (PBL Interferon Source, product # 11100-1, and 11415-1, respectively) at 200 U/mL. The cytokines were applied to the culture medium during the time of initial infection and at the time of each cell passage (every two days). Selection of IFNα- and IFNβ-resistant U87-MG cells were performed by seeding parental U87-MG cells in 12-well plates. Cells were selected in the presence of a high concentration (1000 U/mL) of exogenous IFNα or IPNβ in culture for approximately 21 days. Following 21-day selection in culture, cells were recovered and expanded in the absence of IFNα/β.

Experiments in which U87-MG cells were treated with poly (I:C), cells were seeded the day before transfection at 1 × 10⁵ cells per well in 12-well plates. Cells were then transfected with 10 µg of poly (I:C) (Sigma, cat # P0913) using HD FuGene HD transfection reagent (Roche, cat# 04709691001). Approximately 8 hours post transfection, supernatant was collected for an enzyme linked sandwich assay (ELISA, PBL Interferon Source) in order to quantify the amount of IFNα and IPNβ.

**MLV-based vector production.** Toca 511 and RRV-GFP are Moloney MLV-based RRVs with an amphotropic envelope gene and an EMCV IRES-transgene cassette downstream of the env gene (*e.g*., see Perez OD, et al. Mol Ther 20(9):1689-1698 (2012); U.S. Pat. Nos. 6,410,313, 8,652,460, 8,722,867, and 8,741,279, which describe the sequence and structure of the vector, methods of manufacture, use and infectivity). pBA9b plasmid is a MLV-based replication-defective retroviral vector (*e.g*., see Sheridan PL, et al. Mol Ther 2(3):262-275 (2000)). Expression vectors encoding MLV *gag-pol*, amphotropic envelope gene and VSV-G gene were also utilized. Virus stocks of RRV-GFP and Toca 511 were generated from stable human producer cell lines followed by column purification. Virus stocks of Toca 511 pseudotyped with glycoprotein-G of vesicular stomatitis virus (VSV-G) vector, replication defective MLV-based retroviral vector pseudotyped with 4070A amphotropic envelope protein (MLV-A), retroviral vector pseudotyped with VSV-G (MLV-G), and retroviral vector pseudotyped with amphotropic envelope protein (MLV-A+G), (indicated as Toca511-G, MLV-A, MLV-G, and MLV-A+G, respectively) were produced by transient transfection in 293T cells seeded at 2 × 10⁵ cells per cm². Cells were transfected with plasmid DNA (pAC3-yCD2 and CMV-VSVG for Toca 511-G, CMV-gag-pol, pBA9b, CMV-ampho for MLV-A, CMV-gag-pol, pBA9b, CMV-VSVG for MLV-G, and CMV-gag-pol, pBA9b, CMV-ampho and CMV-VSVG for MLV-A+G) 20 hours post cell seeding using a calcium phosphate method. Eighteen hours post transfection, cells were washed with PBS twice and incubated with fresh complete medium. Viral supernatant was collected approximately 42 hours post transfection and filtered through a 0.45 µm filter. Filtered viral supernatant was subjected to benzonase (Sigma) treatment at 5 U/mL at 4 °C overnight so as to eliminate any contamination by plasmid DNA (which could stimulate TLR9 responses), followed by centrifugation at 18,000 rpm for 250 min. For Toca511-G and MLV-A +G viral particles, high-speed centrifugation purification with a sucrose cushion was performed to remove unincorporated viral glycoproteins. All concentrated viral particles were suspended in formulation buffer.

RRV viral titers were determined as described in Perez OD, et al., Mol Ther 20(9):1689-1698 (2012). Briefly, vector preparations were tittered on PC3 cells by single cycle infection of a dilution series of the vector. The single cycle infection was provided by treatment with AZT, followed by qPCR of target cell genomic DNA specific for integrated viral vector to quantify the number of integrated proviruses. Viral titer reported in transduction unit per milliliter (TU/mL) was determined by calculation of Ct values derived from a standard curve ranging from 1 × 10⁵ copies to 1 × 10¹ copies of plasmid DNA and from known amount of genomic DNA input, number of cells, and dilution factor of viral stock per reaction used in each reaction.

VLPs produced in this study are virus-like particles lacking envelope glycoprotein and viral RNA or virus-like particles lacking envelope glycoprotein, but packaged with pBA9b viral RNA (VLPs/pBA9b). Supernatant from >95% confluent 293GP which express high levels of Moloney MLV gag and pol (Sharma et al., Proc. Natl. Acad. Sci. USA, 94:10803-10808, 1997), or 293GP/pBA9b cells which were transduced with VSV-G pseudotyped MLV vector at MOI of 50 was collected and filtered through a 0.45 µm filter followed by centrifugation at 19,500 × *g* for 30 minutes at 4 °C. All concentrated viral particles were suspended in formulation buffer.

Core VLP production. Core VLP, particles are produced by two methods. A)Enveloped VLP made as described above are treated with dilute detergent to dissolve the membrane without affectng the core protein gagpol particles. Alternatively Core VLP can be made by synthesizing MLV gag protein synthetically or in bacteria and allowing self assembly of core VLP particles (Morikawa op.cit). Activity can be confirmed by testing with plasmacytoid dendritic cells as described below and in the figures.

**Lentiviral vector production.** The HIV-1 based lentiviral vector encoding GFP and pseudotyped with VSV-G (LV-G) was produced by transient transfection in 293T cells as previously described in Dull T, et al., J Virol 72(11):8463-8471 (1998). Viral supernatant was collected 36 hours post transfection followed by benzonase treatment at 5 U/mL overnight at 4 °C. Subsequently, the viral supernatant was concentrated by ultra-centrifugation, and concentrated. The viral stock was resupended in formulation buffer as described in Ostertag D, et al., Neuro Oncol 14(2):145-159 (2012). Viral titer assay was performed by transducing 293T cells with serial 1:10 dilution from the concentrated viral stock in a total volume of 1 mL. Cells were harvested 48 hours post transduction and analyzed by flow cytometry to determine the percentage of GFP positive cells. Viral titer reported as TU/mL was determined by (% GFP-positive cells × number of cells seeded × dilution factor).

**Viral replication monitored by GFP expression**. To monitor viral replication in HT1080, U87-MG, LN-CaP, Sup-T1, WI-38 and primary human umbilical vein endothelial cells (HUVEC), 2 × 10⁵ cells in T25 flask were infected with RRV-GFP at an MOI of 0.01. Every 3-4 days, a portion of cells were passaged with fresh culture medium for continued monitoring of viral replication, and a portion of cells were harvested to determine GFP expression by flow cytometry. Cells harvested for flow cytometry were washed with PBS and centrifuged at 1000 rpm for 5 minutes. Cell pellets were resuspended in PBS containing 1% paraformaldehyde. The percentage of GFP positive cells was determined by flow cytometry using proper gating to exclude GFP-negative cells. Percentage of GFP-positive cells was measured by Canton II using FL1 channel (BD Biosciences). Viral replication kinetics was obtained by plotting percentage of GFP-positive cells over time.

**Viral particle internalization assay**. The same amount of viral particles (Toca 511 and VLPs) used for the pDC bioassay were labeled with CFDA-SE using the Vybrant SFDA-SE Cell Tracer kit (Molecular Probes). The labeled VLP were dialyzed against PBS overnight at 4 °C and added to pDCs. Intracellular fluorescent conjugated CFSE at indicated time points is detected by flow cytometry using the FL-1 channel.

**Deglycosidases, trypsin and phospholipase C treatment of viral particles**. Toca 511 was treated with deglycosidases according to manufacturer's protocol (Promega cat # V493A), with trypsin at 40 µg/mL (Sigma-Aldrich cat # T1426) or with phospholipase C (PLC) at 50 µg/mL (Sigma-Aldrich, cat # P8804) in a total volume of 50 µL. All reactions were incubated for 30 minutes at 37 °C. At the end of incubation, 1 mL of PBS was added to each sample followed by centrifugation at 19,500 × *g* for 30 minutes at 4 °C. The samples were resuspended in 20 µL PBS for immunoblotting or pDC bioassay.

**Relative expression of cellular RNA by qRT-PCR**. RNA was extracted from cells using the RNeasy Kit with DNase I treatment (Qiagen). Reverse transcription was carried out with 100 ng total RNA using High Capacity cDNA Reverse Transcription Kit (ABI). Quantitative PCR (qPCR) was performed using TaqMan Universal PCR Master Mix, No AmpErase UNG (ABI, P/N# 4324018) and Taqman gene expression primers (ABI), (ID# Hs00169345_m1 for EIF2AK2/PKR, Hs00222415_m1 for APOBEC3G, and Hs99999905_m1 for GAPDH) according to manufacturer's protocol. Relative expression of each gene is expressed as 2^{-ΔΔ(Ct)} (see appliedbiosystems.com)

**PCR array expression assay**. RNA was extracted from cells using the RNeasy Kit with DNase I treatment (Qiagen). Reverse transcription was performed using First Strand cDNA Synthesis Kit (Qiagen). qPCR reactions for human IFNα/β response pathway RT² profiler PCR array (Qiagen cat # PAHS-016D) were set up using RT2 SYBR Green Master Mix and carried out according to manufacturer's protocols (Qiagen). Data analysis, scatter plot, fold regulation of each experimental study were obtained using a web-based software provided by Qiagen (see http:]] [[//pcrdataanalysis.sabiosciences.com/pcr/arrayanalysis.php). The fold regulation boundary was set at 2. A fold regulation value greater than 2 indicates an up-regulation, and a fold regulation less than negative 2 indicates a negative or a down-regulation.

**Immunoblotting.** One milliliter of the filtered viral supernatant was concentrated through a 0.4 mL 20% sucrose cushion at 14,000 rpm for 30 minutes. Viral pellets were resuspended in PBS and 2X loading buffer followed by PAGE electrophoresis. Anti-MLV p30, anti-gp70 and anti-VSV-G antibodies were used to detect the expression of MLV capsid protein, envelope protein and VSV-G, respectively. Incorporation of amphotropic envelope protein and/or VSV-G into MLV-based viral particles was detected using anti-MLV p30 (ATCC, cat # CRL-1912) anti-gp70 (83A25) and anti-VSV-G (Abcam, cat # ab50549) antibodies and the corresponding secondary antibodies conjugated with HRP to detect the expression of MLV capsid protein (CA), envelope protein and VSV-G, respectively.

**Isolation of plasmcytoid dendritic cells from human PBMCs and quantification of IFNα**. Buffy coats were obtained from San Diego Blood Bank. PBMCs were purified from the blood of healthy human donors by density gradient centrifugation using Ficoll-Paque (Cellgro, cat # MT-25-072-CVRF). Isolation of pDCs was subsequently enriched by negative selection using magnetic beads (Milteny Biotec, cat # 130-092-207). Cells were maintained in AIM V (Invitrogen cat # 12055-091) in the presence of recombinant IL3 at 10 ng/mL (R & D Systems, cat # 203-IL). The purity of pDCs (> 95%) was confirmed by flow cytometry using anti-CD45 and anti-CD303 antibodies (Miltenyi Biotec cat# 130-080-201 and 130-090-905). Cells were incubated with retroviral vectors at an MOI of 1, 10 or 100 or with lentiviral vector as a positive control at MOI of 40. Approximately 36 hours post infection, supernatant was collected for enzyme linked sandwich assay (ELISA, PBL Interferon Source) to quantify the amount of IFNα. Synthetic oligonucleotide CpG ODN2395 was obtained from InvivoGen (cat # tlrl-odnc). Final concentration of 1 µM was used to activate TLR9 in pDCs.

**Replication kinetics of RRV vector expressing GFP in cultured human tumor cells, non-transformed and primary cells**. FACS analyses and an RRV containing an IRES-GFP cassette downstream of *env* (RRV-GFP) was used to monitor viral spread over time in infected cultured cells. The extent of viral replication of RRV-GFP in a panel of established human tumor cell lines including U87-MG (glioblastoma), HT1080 (fibrosarcoma), LNCaP (prostate), and Sup-T1 (T-cell leukemia) was first examined. In addition, viral replication of RRV-GFP in established primary cells (human non-transformed lung fibroblasts WI-38, and human umbilical vein endothelial cells HUVEC) was examined. RRV-GFP replicated efficiently in all tumor cells (see **FIG. 1A-D**) and, unexpectedly, in non-transformed and primary cells (see **FIG. 1E and 1F**) that have intact Type I IFN signaling pathways. The time required for the vector to reach maximal infectivity varied, ranging from 6 to 28 days, depending somewhat on the rate of cell proliferation, a major determinant for efficient viral spread and productive infection. The replication kinetics of the RRV-GFP was particularly slow in LNCaP and WI-38 cells, which correlates with their slow cell proliferation rate in culture. Nevertheless, the vector reached 80-90% infectivity by day 28 and day 24 post infection, respectively. Together, the data indicated that RRV-GFP replicates in proliferating tumor and primary fibroblast and endothelial cells, *in vitro.*

**Replication of RRV expressing GFP is sensitive to antiviral response induced by exogenous IFNα/β.** For simple retroviruses such as MLV-based RRVs, selectivity towards tumor cells relies, at least in part, on the proliferation of the tumor cells. Although the rate of viral replication does not always correlate with cell proliferation, proliferation is necessary for virus replication. The Type I IFN pathway plays a direct role in regulating antiviral function, but it could also have an antiviral effect that relies solely on its anti-proliferative effect on target cells. To differentiate between the two possible types of antiviral actions, a panel of human tumor-derived cell lines and primary fibroblast cells (WI-38) was examined in order to determine whether the cells were sensitive to antiviral response by exogenous IFNα/β. The basal and IFNα-induced expression of the genes for two proteins, PKR and APOBEC3G, was first compared. PKR is an IFNα/β-responsive gene commonly used for monitoring the activation of the Type I IFN pathway and APOBEC3G has been shown to inhibit replication of retroviruses. Cells were treated with exogenous IFNα (at 200 U/mL), harvested at 8, 16 and 24 hours post treatment and gene expression measured by qRT-PCR. PKR and APOBEC3G gene expression were induced as early as 8 hours and sustained up to 24 hours post treatment with exogenous IFNα (see **FIG. 2A** and **2B**). The responsiveness to exogenous IFNα was highest in non-transformed WI-38 cells, intermediate in HT1080 and U87-MG cells, and lowest in 293T cells.

Further experiments were then performed to determine whether replication of the RRV-GFP was sensitive to exogenous IFNα/β treatment. U87-MG cells were selected for the experiments due to rapid viral reproduction in this particular cell line, possibly due to some defect in the Type I IFN pathway. As shown in **FIG. 2C** and 2D, viral replication was completely inhibited by concomitant addition of IFNβ, whereas residual viral replication was observed in cells treated with IFNα. Taken together, the data indicate that HT-1080 and U87-MG tumor cells were responding to exogenous Type I IFNα/β, although the levels of IFN responsive gene induction appear reduced in these cells compared to that in WI-38 fibroblasts. Further, in U87-MG cells, the IFN-mediated antiviral response resulted in remarkable attenuation of viral replication.

**Selection and characterization of IFNα- and IFNβ-resistant cells.** As evasion of innate immunity can lead to efficient viral replication, it was reasoned that selection of Type I IFN-resistant cells from a pool of IFN-responsive tumor cells may further enhance viral replication within the tumor. IFNα- and IFNβ-resistant U87-MG cells were selected and investigated to determine whether RRV-GFP would replicate more efficiently in the resistant cells. Following 21-day selection in culture, cells were recovered and expanded in the absence of IFNα/β. Subsequently, resistant cells were infected with RRV-GFP in the absence of exogenous Type I IFN to examine whether viral replication was more efficient in IFN-resistant cells than in the parental cells. The results showed that in the absence of exogenous IFNα/β, IFNα-resistant cells (U87IRA) exhibited a significant attenuation in viral spread compared to the IFN-sensitive parental cells, whereas IFNβ-resistant cells (U87IRB) exhibited a marginal attenuation in viral spread (see **FIG. 3A** and **3B**). As previously noted, attenuation of viral spread could be associated with changes in cell proliferation, as the resistant cell lines do proliferate more slowly than the parental U87-MG line.

To examine the function of Type I IFN-mediated antiviral response in IFNα- and IFNβ-resistant cells, the replication kinetics of RRV-GFP were determined in the presence of exogenous IFNα and IFNβ. As shown in **FIG. 3C**, the spread of RRV-GFP in the presence or absence of IFNα was comparable in the IFNα-resistant cells. In contrast, RRV-GFP appears to spread more efficiently in the presence of IPNβ during the early stage of infection in the IFNβ-resistant cells (see **FIG. 3D**). The data is in contrast to findings in the parental cells, in which spread of RRV-GFP was strongly inhibited in the presence of exogenous IFNα/β (see **FIG. 2**). Furthermore, resistance to the action of exogenous IFNα/β by attenuation of the signaling pathway was confirmed by showing lack of IFN-inducible gene expression of PKR and APOBEC3G (see **FIG. 3E-F**) and the Type I IFN pathway-specific genes by PCR array (see **TABLE 1**). Collectively, the data demonstrate that the cells become insensitive to exogenous IFNα/β, although viral replication in the absence of exogenous IFNα/β was not enhanced in these cells probably due to co-selection of slow proliferating cells.

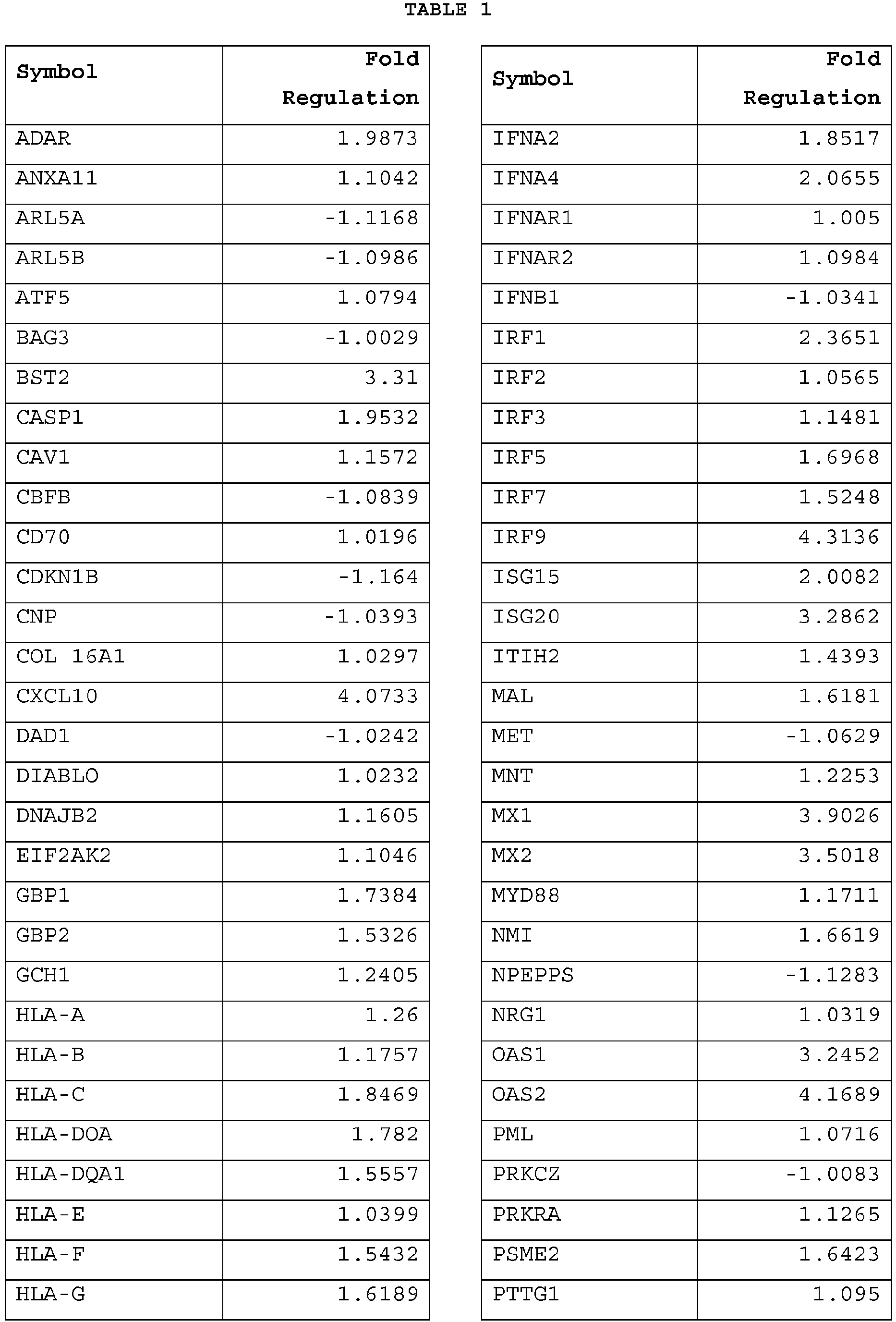

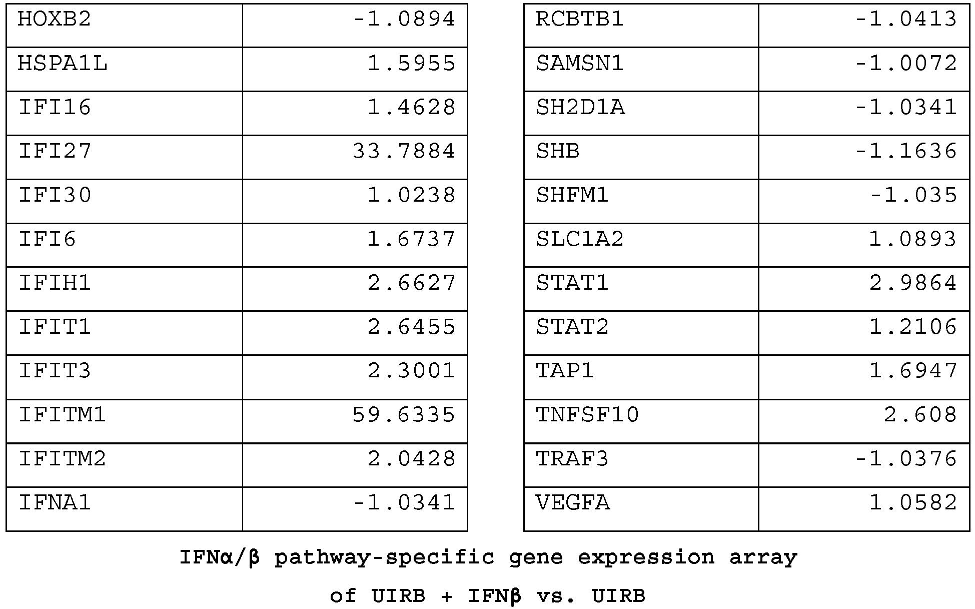

**Toca 511 RRV does not trigger IFNα/β response in U87-MG cells.** The above findings suggest that RRV-GFP upon entry or during its life cycle does not directly trigger IFNα/β responses in the host cells. To confirm these results from the RRV-GFP were not vector- or transgene-specific, IFNα/β response to Toca 511 was assessed by measuring pathway-specific gene expression in U87-MG cells infected with Toca 511 at MOI of 10. At 16 hours post Toca 511 infection, or IFNβ treatment, cells were harvested for gene expression analysis by PCR arrays. Several Type I IFNα/β inducible genes including Bst2, CXCL10, PKR, IFI and IFITM, ISG, Mx and OAS in cells treated with exogenous IFNβ were upregulated by more than 100-fold compared to those of the untreated (See **FIG. 6A** and **TABLE 2**).

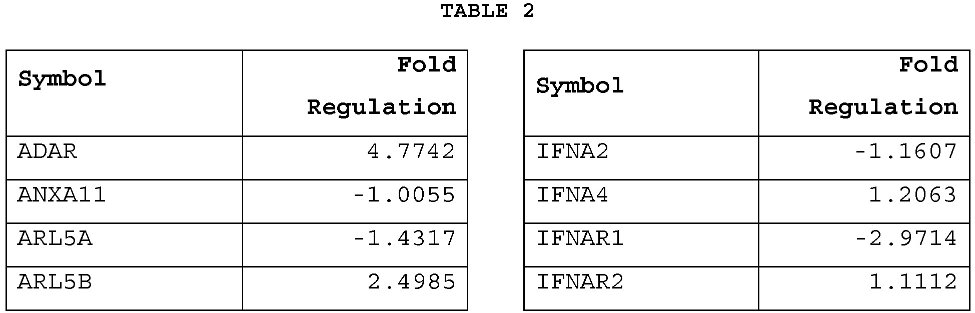

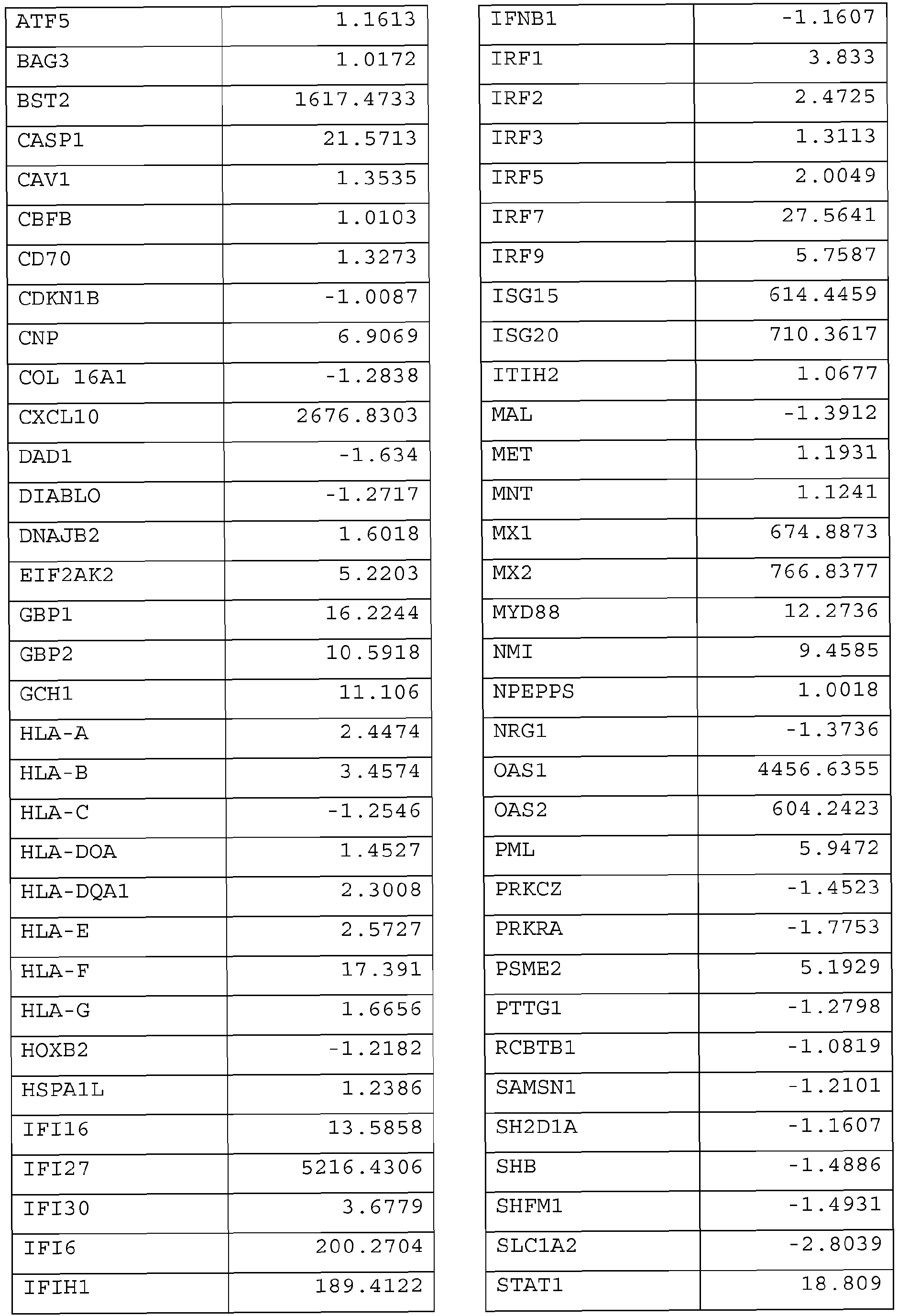

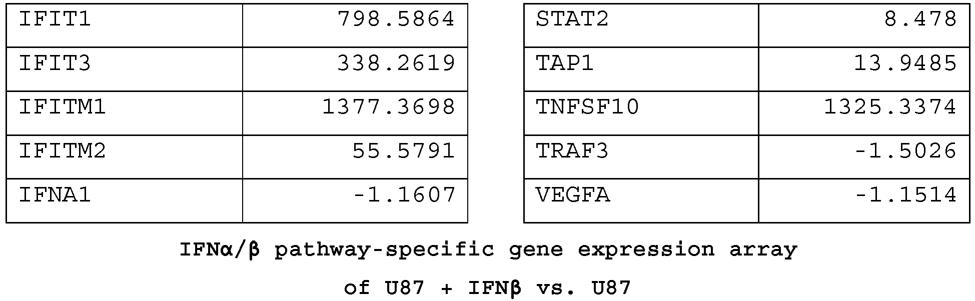

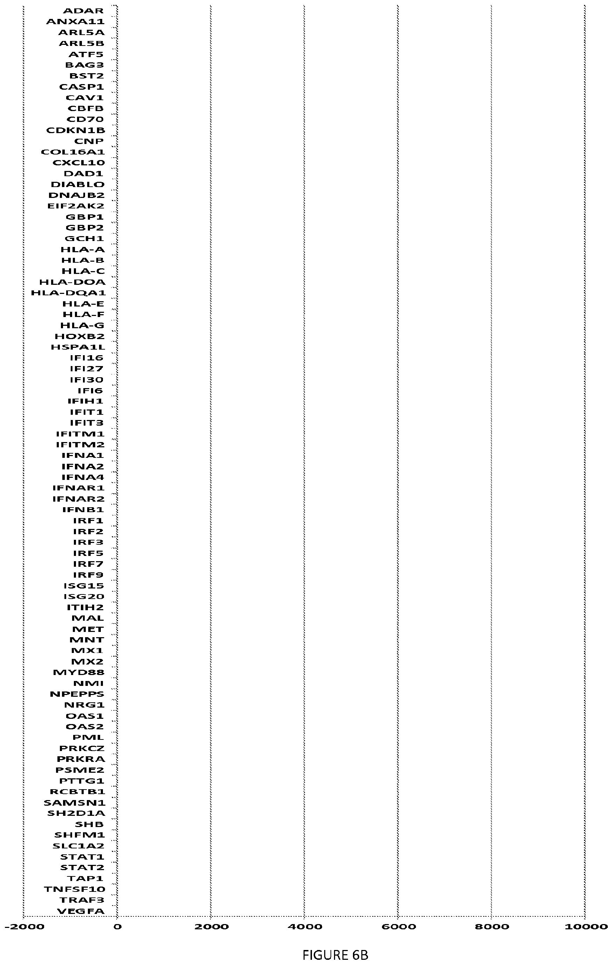

In contrast, there was no global induction of the IFNα/β inducible genes observed in cells infected with Toca 511 (see **FIG. 6B** and, **TABLE 3**).

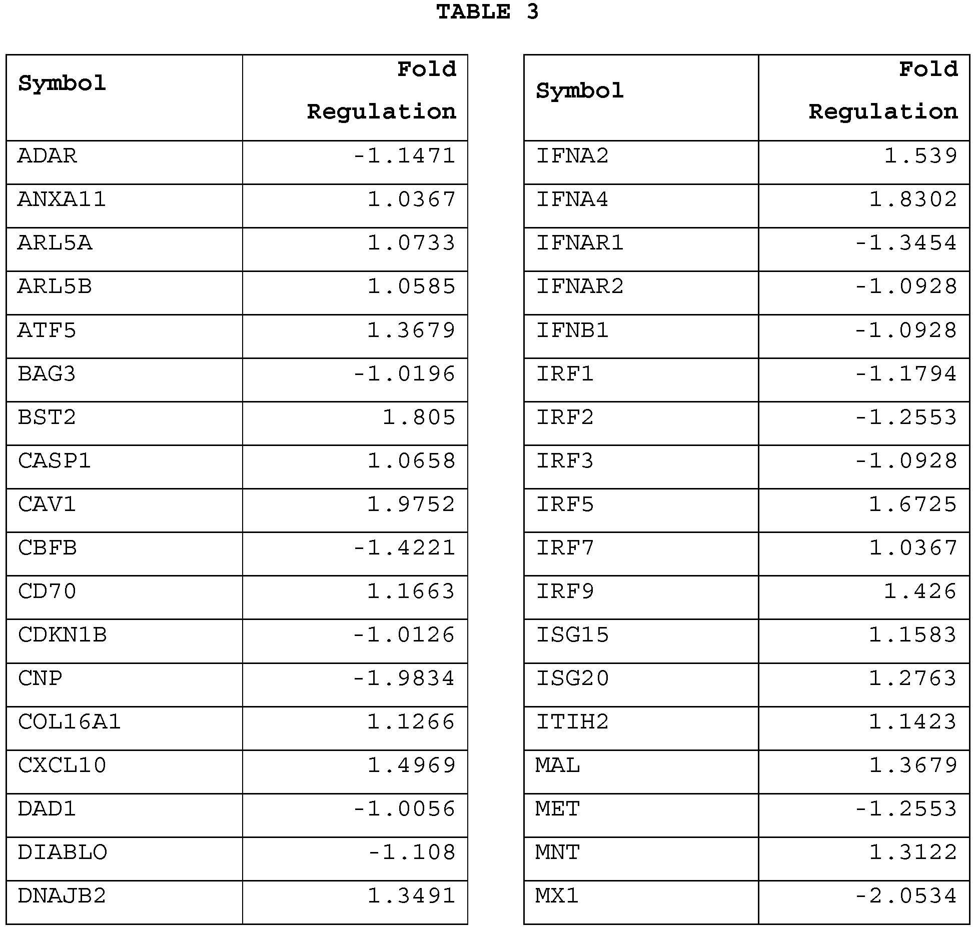

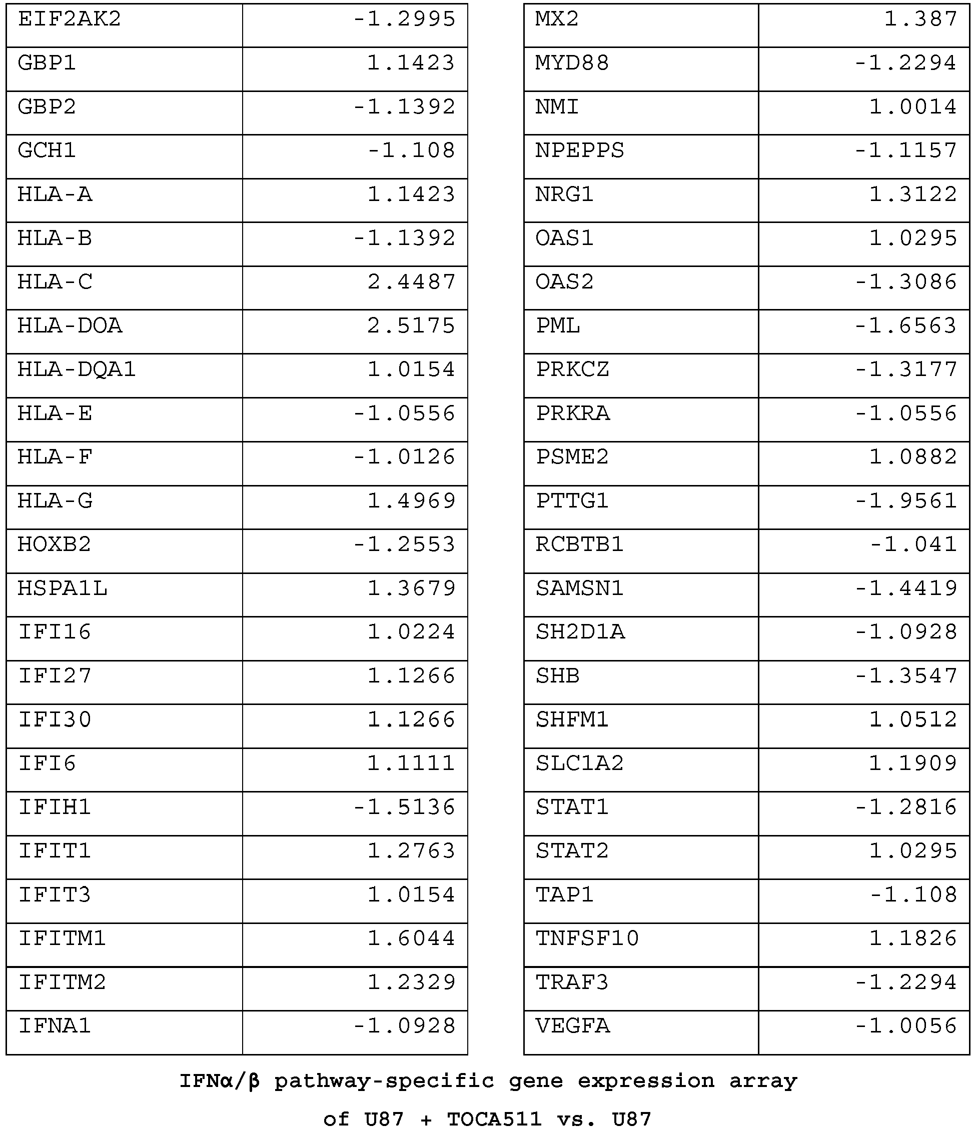

In addition to the viral replication data, the gene expression PCR array data further support the notion that simple MLV-based RRVs do not directly trigger Type I IFN response in IFN-responsive U87-MG tumor cells. As the viral genome of RRV (*e.g*. Toca 511) comprises two identical single-stranded RNA copies with extensive secondary structures, the nucleic acid component presumably can serve as a potential PAMP motif during its life cycle in the host cells. It is possible that U87-MG cells have a functional TLR pathway but are deficient in IFNα/β production due to deletions and rearrangement in the IFN gene cluster located at chromosome 9p21-22.

To further examine the interaction of TLR and Type I IFN signaling pathways and to delineate the mechanism supporting the permissiveness of Toca 511 replication in U87-MG cells, U87-MG cells were tested to see if the cells were capable of producing IFNα/β upon activation of TLR3, which is expressed in most cells and serves as a key sensor for viral dsRNA. U87-MG cells were treated with Toca 511 or with poly (I:C) (agonist for TLR3, RIG-1 and MDA5) as a positive control. The same experiment was performed with human dermal primary fibroblasts CCD-1070Sk, which support the replication of the RRV-GFP (see **FIG. 7**). Fibroblasts are key IFNβ-producing cells. As shown in **FIG. 4A**, IFNβ production was significantly induced in both cell lines treated with poly (I:C), whereas IPNβ production was undetectable when treated with Toca 511 at 8, 16 and 24 hours post infection, respectively. In contrast, IFNα expression was detected in neither CCD-1070Sk nor U87-MG cells treated with poly (I:C) or Toca 511 (see **FIG. 4B**). The absence of IFNα expression as a result of secondary induction by IFNβ in CCD-1070Sk and in U87-MG cells was likely due to the sample collection before IFNα production, although in U87-MG cells the absence of IFNα induction could also be due to deletion of the IFNα gene cluster in chromosome 9p21-22.

Altogether, the data indicates that U87-MG supports replication of MLV-based RRV even when both TLR (IFN production) and Type I IFN signaling pathways remain at least partially functional in these cells. One possible explanation for the results could be the absence or camouflaging of potential ligands for TLR3 in RRVs.

**An active immunosuppressive component associated with Toca 511 blocks IFNα/β response in human pDCs.** The possibility that RRV may trigger other PRRs such as TLR7 and TLR9 and that this could occur *in vivo* in non-malignant cells within the tumor microenvironment could not be excluded. Therefore, the antiviral response of human primary plasmacytoid dendritic cells to RRV was examined (a component of the tumor microenvironment in some tumor types).

pDCs are a subset of human dendritic cells that differentially express TLR7 and TLR9. As with lentiviral vectors, the duplicates of single-stranded RNA viral genomes encapsulated in the RRV viral particles and unmethylated double-stranded DNA that arises from reverse transcription inside the viral particles could potentially serve as PAMP motifs for TLR7 and TLR9, respectively. The sequence of Toca 511 for TLR-activating GU-rich sequences and identified several GU-rich motifs (ranging from 60-80%) of 20-nucleotide length located in the psi region of the viral genome was examined (See **FIG. 8**).

To investigate whether the viral genome of Toca 511 could potentially be a PAMP motif for TLR7 in human pDCs, pDCs were isolated from PBMCs of healthy individuals and exposed to Toca 511 at MOI of 1, 10 and 100. These cells do not replicate and therefore a productive viral infection would not be expected. In the experiments, ODN2395 (agonist for TLR9) and lentiviral vector pseudotyped with vesicular stomatitis virus glycoprotein, LV-G (agonist for TLR7), were included as positive controls for induction of IFNα production in pDCs. The data demonstrate that Toca 511, even at high MOIs, did not induce IFNα production in human pDCs, whereas a significant level of IFNα production was detected in cells treated with ODN2395 and LV-G (see **FIG. 5A**). The production of IFNα by pDCs treated with LV-G demonstrates that transduction of murine pDCs with a lentiviral vector pseudotyped with vesicular stomatitis virus glycoprotein induced IFNα production. Furthermore, the pDCs experiments were performed using mouse pDCs preparations exposed to RRV and LV-G, with results essentially identical to those shown here with the human pDCs preparations (see **FIG. 9**).

Next, the possibility that the viral glycoprotein involved in cell entry plays a role in determining the subcellular localization of the viral particle upon entry and their exposure to cellular immune sensors was explored. To this end, Toca 511 was generated both with amphotropic and VSV-G proteins on the viral particles in order to redirect Toca 511 to the endocytic pathway. As shown in **FIG. 5B**, both amphotropic *env* and VSV-G proteins were efficiently incorporated into viral particles, with a slight decrease of the amphotropic *env* incorporation when VSV-G was co-expressed. In addition, by qPCR, the function of VSV-G in directing viral entry and one-round transduction, resulting in the integration of proviral DNA into host genome, was confirmed using CHO-K1 cells, which are permissive for VSV-G pseudotyped RRV but not for amphotropic enveloped RRV. Toca 511 pseudotyped with VSV-G which directs the viral particles preferentially towards the endocytic pathway could trigger the activation of endosomal TLRs (TLR7/8 or TLR9) was then examined. Cultured human pDCs were treated with Toca 511 or Toca 511 co-enveloped with VSV-G. Consistent with previous data, Toca 511 by itself did not stimulate human pDCs to produce IFNα, Toca 511 co-enveloped with VSV-G also did not activate endosomal TLRs in human pDCs (see **FIG. 5C**).

**Immunosuppressive component associated with Toca 511 can be abrogated by heat inactivation to trigger IFNα/β response in human pDCs.** One explanation for the finding that Toca 511 does not trigger Type IFN response in human pDCs is that an immunosuppressive component is associated with RRV particles. To investigate whether the classic viral inactivation method by heat would inactivate a suppressive component and therefore allow the induction of IFNα/β expression, Toca 511 was treated with heat at 56 °C for 30 minutes prior to exposure to pDCs in culture. The data revealed that in contrast to untreated Toca 511, heat-inactivated Toca 511 induced marked IFNα production by pDCs (see **FIG. 5D**). A similar result was obtained when Toca 511 co-enveloped with VSV-G was treated with heat inactivation (**FIG. 5D**). The data provide evidence that an active immunosuppressive component is associated with Toca 511 and it can be inactivated by heat treatment.

Experiments were then performed where heat-inactivated Toca 511 was co-incubated with untreated Toca 511 or with lentiviral vector at 1:1 ratio (transduction unit) prior to exposure to pDCs. The data indicated that co-incubation of Toca 511 with heat-inactivated Toca 511 or with lentiviral vector pseudotyped with VSV-G resulted in blockade of IFNα production in human pDCs (see **FIG. 5E**). Together, the data indicates that there is an active immunosuppressive component associated with viral particles and that heat inactivation of viral particle abrogates the immunosuppressive function, which leads to production of IFNα in pDCs. The heat inactivation data also indicate that productive infection is not required for activation of cellular innate responses in pDCs.

**Immunosuppressive component associated with Toca 511 is not due to the presence of the amphotropic envelope glycoprotein.** To examine whether the immunosuppressive component resides in the amphotropic envelope glycoprotein, 3 replication-defective retroviral vectors pseudotyped with the amphotropic (MLV-A), VSV-G glycoprotein (MLV-G) or amphotropic envelope and VSV-G glycoproteins(MLV-A + G) were generated. The viral RNA genome (pBA9b) used in generating these vectors shares 100% sequence homology with Toca 511 in the psi region, in which several GU-rich sequence motifs are present (see **FIG. 8**). Consistent with previous data, none of the 3 vectors stimulated pDCs to produce IFNα (see **FIG. 5F**). However, when these vectors were subjected to heat inactivation, all 3 vectors stimulated pDCs to produce IFNα (see **FIG. 5G**). Collectively, the data indicated that an immunosuppressive component associated with MLV-based vectors blocks the activation of endosomal TLRs in pDCs and subsequent induction of Type I IFN production and that the function of the immunosuppressive component associated with viral particles (Toca 511, Toca 511-G, MLV-A, MLV-G, and MLV-A + G) is a dominant active process. More importantly, the lack of IFNα production in pDCs exposed to the replication-defective vector pseudotyped with VSV-G glycoprotein indicates that the immunosuppressive component associated with the viral particles was not attributable to the amphotropic enveloped glycoprotein.

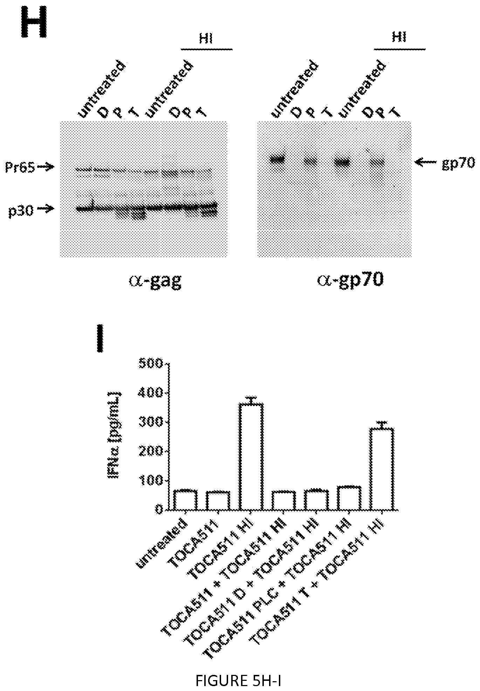

To investigate whether the component is sensitive to enzyme treatment that can alter the protein components of the viral particles, the effect of deglycosidases, PLC and trypsin were examined on pDC stimulation. As seen in **FIG. 5H**, the electrophoresis pattern of the gag polyprotein from heat-treated Toca 511 was comparable to that of non-heat-treated. Co-incubation of deglycosidases, PLC or trypsin with Toca 511 further alters the electrophoresis pattern of the gag polyprotein and/or the envelope protein. For example, both deglycosidases and trypsin treatment resulted in change in gag polyprotein intensity and decrease in molecular size and/or disappearance of the gp70 band. Toca 511 was treated with deglycosidases, PLC or trypsin and co-incubated with heat-treated Toca 511 prior to exposure to pDCs in culture. The data revealed co-incubation of trypsin-treated, but not deglycosidase- or PLC-treated Toca 511 with heat-treated Toca 511 abrogated the dominant immunosuppressive activity of Toca 511 and partially induced IFNα production in pDCs (**FIG. 5I**).

Together, the data indicate that there is an active immunosuppressive component associated with Toca 511 viral particles and that heat or trypsin treatment of Toca 511 viral particle abrogates the immunosuppressive function, which leads to production of IFNα in pDCs. The trypsin data further strengthen the notation that the immunosuppressive component is a protein component. The data also indicate that productive infection is not required for activation of cellular innate responses in pDCs.

**Immunosuppressive component associated with Toca 511 is not due to the presence of the glyco-gag nor the amphotropic envelope glycoprotein.** The glyco-gag (Pr80gag) expressed from retroviruses has shown to be incorporated into viral particles and plays a role in inhibiting the function of APOBEC3 in target cells. In addition, an immunosuppressive peptide located in the TM subunit of several MLV envelope proteins has been described. To ask where the immunosuppressive component resides in the viral particles, 3 retroviral non-replicating vectors pseudotyped with the amphotropic (MLV-A), VSV-G glycoprotein (MLV-G) or amphotropic envelope and VSV-G glycoproteins (MLV-A+G) that do not express glyco-gag were generated. The viral RNA (pBA9b) used in generating these vectors shares 100% sequence homology with Toca 511 in the *psi* region, in which several GU-rich sequence motifs are present. Consistent with previous data, none of the 3 vectors stimulated pDCs to produce IFNα (**FIG. 10A**). However, when these vectors were subjected to heat treatment, all 3 vectors stimulated pDCs to produce IFNα (**FIG. 10B**).

To further examine whether the envelope glycoprotein could be part of the immunosuppressive component, non-enveloped VLPs packaged with pBA9b (VLPs/pBA9b) or without pBA9b vector (VLPs) were generated. Consistent with observed data, VLPs and VLPs/pBA9b did not induce IFNα production in pDCs, whereas heat-treated VLPs and VLPs/pBA9b did (**FIG. 10C**). Collectively, the data indicate that an immunosuppressive component associated with MLV-based viral particles blocks Type I IFN production in pDCs and that the function of the immunosuppressive component associated with viral particles (Toca 511, Toca 511-G, MLV-A, MLV-G, and MLV-A+G) is a dominant active process. In addition, the inability of MLV-A, MLV-G, and MLV-A+G vectors to stimulate IFNα production in pDCs in the absence of glyco-gag suggests that glyco-gag is not part of the immunosuppressive component. Furthermore, the immunosuppressive function is likely not attributable to the amphotropic enveloped glycoprotein. Finally, the ability of VLPs without pBA9b viral RNA and after heat treatment to induce IFNα production in pDCs also suggests that there is possibly another PAMP, in addition to the viral RNA, present in the viral particles.

**Internalization of Toca 511 and VLPs by pDCs.** The ability of VLPs carrying or lacking the pBA9b viral RNA to induce IFNα production in pDCs suggest that the PRR may be located on the cell surface as VLPs are not expected to be internalized efficiently in the absence envelope protein. The capacity of freshly isolated immature pDCs to internalize Toca 511 and VLPs lacking the pBA9b viral RNA using CFSE-labeled Toca 511 and VLPs was examined. **FIG. 10D** shows that both Toca 511 and VLPs (heat or non-heat treated) were rapidly internalized by pDC as measured by the mean fluorescent intensity (MFI), suggesting that the internalization process in pDC is independent of the Pit-2 receptor. Nevertheless, internalization of heat-treated VLPs was always less efficient. Interestingly, a decrease in MFI was observed over a course of 6 h post incubation with the exception of heat-treated VLPs. One possible explanation is viral protein turnover after internalization. Given that both Toca 511 and VLPs can both be internalized by pDCs, the cellular compartment of the unidentified PRR is not limited to the cell surface.

**Toca 511-associated non-viral nucleic acid PAMP is sensitive to deglycosidases, trypsin and PLC treatment.** To further characterize the unidentified non-viral nucleic acid PAMP associated with Toca 511, sensitivity to enzyme treatment that can alter the protein, but not the nucleic acid, components of the viral particles was examined. The effect of deglycosidases, PLC and trypsin on pDC stimulation was examined. As seen in **FIG. 10E**, the alterations of the viral proteins shown in **FIG. 5H** correlate with the loss of its ability to induce IFNα production in pDCs after heat treatment. In contrast to the immunosuppressive component associated with Toca 511 which is sensitive only to trypsin, the immune-stimulating component associated with Toca 511 viral particles is sensitive to deglycosidases, PLC and trypsin. All together, the data suggest that both the immune-stimulating and immune-suppressive components are likely structural elements and, therefore, viral protein-based, but, in the case of the immune-stimulating component, also involving lipid and/or carbohydrate components.

## Claims

1. A composition comprising a non-enveloped, non-replicating viral-like particle (VLP) comprising a naked viral particle consisting of (i) structural gag and pol polypeptides from a gammaretrovirus; and/or (ii) a gamma retroviral gag-pol polyprotein, wherein the VLP lacks a viral genome, a lipid envelope, and envelope proteins.

2. The composition of claim 1, wherein the VLP comprises one or more components associated with a gammaretrovirus that block Type I IFN production.

3. The composition of claim 2, wherein the immunosuppressive components block the activation of endosomal Toll-like receptors (TLRs) in pDCs and subsequent induction of Type I IFN production.

4. A composition comprising a VLP of any of claims 1-3 and further comprising an additional immunosuppressive agent.

5. The composition of claim 4, wherein the additional immunosuppressive agent is an anti-IFNα antibody or fragment thereof.

6. The composition of claim 4, wherein the additional immunosuppressive agent is a steroid.

7. The composition of any of claims 1-5, for use in therapy.

8. The composition of any of claim 1-4 for use in the treatment of a type I interferon mediated autoimmune disorder selected from the group consisting of rheumatoid arthritis, systemic lupus erythematosus, scleroderma, polymyositis, dermatomyositis, ankylosing spondylitis, and Sjogren's syndrome.

9. A composition for use according to claim 8, wherein the immune disorder is systemic lupus erythematosus.

## Patentansprüche

1. Zusammensetzung, die ein nicht umhülltes, nicht replizierendes, virusähnliches Partikel (VLP) umfasst, das ein nacktes virales Partikel umfasst, das aus Folgenden besteht: (i) strukturellen gag- und pol-Polypeptiden von einem Gammaretrovirus; und/oder (ii) einem gammaretroviralen gag-pol-Polyprotein, wobei dem VLP ein virales Genom, eine Lipidhülle und Hüllproteine fehlen.

2. Zusammensetzung nach Anspruch 1, wobei das VLP eine oder mehrere Komponenten, die mit einem Gammaretrovirus verbunden sind, umfasst, die die Produktion von Typ-I-IFN blockieren.

3. Zusammensetzung nach Anspruch 2, wobei die immunsuppressiven Komponenten die Aktivierung von endosomalen Toll-like Rezeptoren (TLRs) in pDCs und anschließende Induzierung der Produktion von Typ-I-IFN blockieren.

4. Zusammensetzung, die ein VLP nach einem der Ansprüche 1-3 umfasst und weiter ein zusätzliches Immunsuppressivum umfasst.

5. Zusammensetzung nach Anspruch 4, wobei das zusätzliche Immunsuppressivum ein Anti-IFNα-Antikörper oder Fragment davon ist.

6. Zusammensetzung nach Anspruch 4, wobei das zusätzliche Immunsuppressivum ein Steroid ist.

7. Zusammensetzung nach einem der Ansprüche 1-5 für die Verwendung in der Therapie.

8. Zusammensetzung nach einem der Ansprüche 1-4 für die Verwendung bei der Behandlung einer Typ-I-Interferon-vermittelten Autoimmunerkrankung, die aus der Gruppe ausgewählt ist, die aus rheumatoider Arthritis, systemischem Lupus erythematodes, Sklerodermie, Polymyositis, Dermatomyositis, ankylosierender Spondylitis und Sjögren-Syndrom besteht.

9. Zusammensetzung für die Verwendung nach Anspruch 8, wobei die Immunerkrankung systemischer Lupus erythematodes ist.

## Revendications

1. Composition comprenant une particule pseudo-virale (VLP) non réplicative non enveloppée comprenant une particule virale nue consistant en (i) des polypeptides structurels gal et pol d'un gamma-rétrovirus ; et/ou (ii) une polyprotéine gag-pol gamma-rétrovirale, où la VLP est exempte d'un génome viral, d'une enveloppe lipidique et de protéines d'enveloppe.

2. Composition de la revendication 1, où la VLP comprend un ou plusieurs composants associés à un gamma-rétrovirus qui bloquent la production d'IFN de Type I.

3. Composition de la revendication 2, où les composants immunosuppresseurs bloquent l'activation de récepteurs de type Toll (TLR) endosomaux dans les cellules dendritiques plasmacytoïdes (pDC) et l'induction de la production d'IFN de Type I qui s'ensuit.

4. Composition comprenant une VLP de l'une quelconque des revendications 1-3 et comprenant en outre un agent immunosuppresseur supplémentaire.

5. Composition de la revendication 4, où l'agent immunosuppresseur supplémentaire est un anticorps anti-IFNα ou un fragment de celui-ci.

6. Composition de la revendication 4, où l'agent immunosuppresseur supplémentaire est un stéroïde.

7. Composé de l'une quelconque des revendications 1-5 pour une utilisation en thérapie.

8. Composition de l'une quelconque des revendications 1-4 pour une utilisation dans le traitement d'une maladie auto-immune sous la médiation de l'interféron de Type I sélectionnée dans le groupe consistant en les suivantes : polyarthrite rhumatoïde, lupus érythémateux aigu disséminé, sclérodermie, polymyosite, dermatomyosite, spondylarthrite ankylosante et syndrome de Sjögren.

9. Composition pour une utilisation selon la revendication 8, où la maladie immune est le lupus érythémateux aigu disséminé.
